# EUROPÄISCHE PATENTSCHRIFT

(11) **EP 1 664 174 B1**
(45) Veröffentlichungstag und Bekanntmachung des Hinweises auf die Patenterteilung: **30.05.2007**
(21) Anmeldenummer: 04764165.9
(22) Anmeldetag: 16.08.2004
(51) Int. Cl.: C08K 5/06, C07C 43/307

(54) **AROMATISCHE FORMALE ALS ADDITIVE ZUR ABSENKUNG DER WASSERAUFNAHME VON POLYCARBONATEN**
AROMATIC FORMALS USED AS ADDITIVES FOR REDUCING THE WATER ABSORPTION OF POLYCARBONATES
FORMALS AROMATIQUES UTILISES COMME ADDITIFS POUR REDUIRE L'ABSORPTION D'EAU DE POLYCARBONATES

(30) Priorität: 23.08.2003 DE 10338909
(43) Veröffentlichungstag der Anmeldung: 07.06.2006
(73) Patentinhaber: Bayer MaterialScience AG, 51368 Leverkusen (DE)
(72) Erfinder: HEUER, Helmut-Werner, 47829 Krefeld (DE); WEHRMANN, Rolf, 47800 Krefeld (DE); BRUDER, Friedrich-Karl, 47800 Krefeld (DE)
(86) Internationale Anmeldenummer: PCT/EP2004/009173
(87) Internationale Veröffentlichungsnummer: WO 2005/021629

(56) Entgegenhaltungen:
- EP-A- 0 054 488
- GB-A- 1 116 589
- US-A1- 2002 035 207
- DATABASE CA [Online] CHEMICAL ABSTRACTS SERVICE, COLUMBUS, OHIO, US; TANIMOTO, SHIGEO ET AL: "A facile preparation of benzaldehyde diaryl acetals" XP002307253 gefunden im STN Database accession no. 90:6036 & BULLETIN OF THE INSTITUTE FOR CHEMICAL RESEARCH, KYOTO UNIVERSITY , 56(3), 101-3 CODEN: BICRAS; ISSN: 0023-6071, 1978,

## Beschreibung

Die Anmeldung betrifft neue aromatische Formale, thermoplastische Formmassen enthaltend Polycarbonat und mindestens ein erfindungsgemäßes aromatisches Formal als Additiv zur Absenkung der Wasseraufnahme des Polycarbonates und der Verbesserung der Fließfähigkeit sowie die Verwendung solcher Formmassen zur Herstellung von Formkörpern, insbesondere optischen Datenträgern, wie z.B. Compact Disks, Video Disks, Digital Versatile Disks und weitere ein- oder mehrfach beschreib- wie löschbare optische Datenträger, sowie die entsprechenden Formkörper selbst.

Polycarbonate werden generell wegen ihrer besonderen Eigenschaftskombination wie Transparenz, Wärmeformbeständigkeit und Dimensionsstabilität als Materialien für den Spritzguss bzw. das Spritzprägen von optischen Datenträgern eingesetzt. Zur Verbesserung der Verarbeitbarkeit, die im allgemeinen bei Temperaturen im Bereich von 300°C bis 400°C stattfindet, werden dem Polycarbonat in der Regel Additive, wie Entformungsmittel und Stabilisatoren zugesetzt.

Aromatische Polycarbonate auf Basis von Bisphenol A werden insbesondere für die Herstellung von optischen Datenträgern verwendet. Sie können jedoch bis zu 0,34 Gew % Wasser aufnehmen, was sich ungünstig auf die Dimensionsstabilität der Datenträger auswirken kann. Eine verbesserte Dimensionsstabilität ist jedoch besonders beim Einsatz von blauen bzw. blau-grünen Lasern von Bedeutung.

In US B 6,391,418 werden Substrate für Datenträgermedien beschrieben, welche ein Biphenylderivat als Additiv zur Erhöhung der Dimensionsstabilität (geringerer Schrumpf) enthalten.

In M. Ueda, Mitsubishi Engineering Plastics Corp., Technical Digest of Joint ISOM / ODS 2002 Waikoloa Hawaii, 8. 7. 2002, Page 33 - 35, wird die Zugabe von kleinen Mengen an m-Terphenyl zu Bisphenol A Polycarbonat beschrieben, was zu einer Reduzierung der Wasseraufnahme führt. Der Nachteil dieser Biphenylderivate besteht jedoch darin, dass sie hoch-konjugierte aromatische π-Systeme darstellen, die bereits im blauen bzw. blau-grünen Spektralbereich absorbieren. Dies ist unerwünscht bei Speichertechnologien, welche in diesem Wellenlängenbereich arbeiten. Außerdem stellen Terphenyle relativ starre Moleküle dar, was sich negativ auf die mechanischen Eigenschaften in der Mischung mit Polycarbonat auswirkt.

Die im Stand der Technik beschriebenen Möglichkeiten führen also nicht zu in jeder Hinsicht befriedigenden Ergebnissen. Es ist im Stand der Technik aber auch keinerlei Andeutung zu finden, dass Formale als Additive geeignet sein könnten.

Es bestand daher die Aufgabe, thermoplastische Formmassen, enthaltend Polycarbonat mit einer verringerten Wasseraufnahme bereitzustellen, welche dadurch eine bessere Dimensionsstabilität aufweisen. Insbesondere erfordern die neuen Diskformate mit höherer Speicherkapazität und ggf. geringerer Diskdicke, wie z.B. die Digital Versatile Disks (DVDs), eine höhere Thermostabilität im Vergleich zur CD. Auftretende Materialschädigung bei der Verarbeitung zu Formkörpern und Belagsbildung im Werkzeug werden kritischer. Somit ist es wünschenswert, dass ein Additiv zur Reduktion der Wasseraufnahme gleichzeitig eine Absenkung der Schmelzviskosität und damit ein besseres Fließen bei etwas niedrigeren Temperaturen bewirkt.

Mit den erfindungsgemäßen Formmassen wird diese Aufgabe überraschenderweise durch eine verbesserte Qualität der Datenspeicher und eine verbesserte Verarbeitbarkeit des Materials im Spritzguss bzw. Spritzprägeverfahren und eine reduzierte Wasseraufnahme und damit letztendlich eine verbesserte Dimensionsstabilität gelöst.

Gegenstand der vorliegenden Anmeldung sind daher thermoplastische Formmassen enthaltend mindestens ein Polycarbonat und mindestens ein erfindungsgemäßes aromatisches Formal mit spezieller chemischer Struktur als Additiv zur Reduktion der Wasseraufnahme. Diese aromatischen Formale führen somit zu einer verbesserten Dimensionsstabilität der Datenträger und bewirken gleichzeitig eine niedrigere Schmelzviskosität.

Die Herstellung von aromatischen Polyformalen kann im Unterschied zu Polycarbonat in homogener Phase aus Bisphenolen und Methylenchlorid in Gegenwart von Alkalihydroxiden erfolgen. Methylenchlorid fungiert bei dieser Polycondensation gleichzeitig als Reaktand und als Lösungsmittel. In US B 4,374,974 ist ein Verfahren beschrieben, in dem, ausgehend von speziellen Bisphenolen, nach Umsetzung mit Methylenchlorid, lineare und cyclische Oligo- und Polyformale erhalten werden können. Die Umsetzung von monofunktionellen Phenolen zu niedermolekularen aromatischen Formalen mittels dieser Synthesevariante wird im Stand der Technik nicht beschrieben.

In S. Tanimoto et al., Bull. Inst. Chem. Res., Kyoto Univ., Vol. 56, No. 6, 1978 wird lediglich für bestimmte Methyl- Chlor-, Brom- und Methoxy-substituierte Spezies eine Synthese in DMSO bzw. Acetonitril in Gegenwart von 18-Krone-6 als neuer Einsatzmöglichkeit für diesen Phasentransferkatalysator offenbart. Über die Synthese weiterer Formale oder gar deren Verwendbarkeit wird jedoch nichts gesagt.

Die erfindungsgemäßen aromatischen Formale basieren auf der allgemeinen Formel 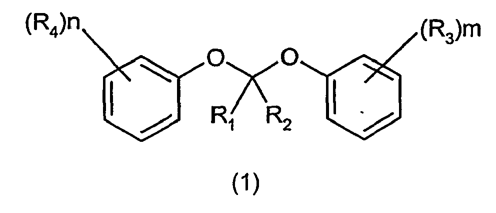 worin
R₁ und R₂ für Wasserstoff oder Phenyl,
R₃ und R₄ unabhängig von einander für Wasserstoff, lineare oder verzweigte C₁-C₄₀-Alkyl oder Alkoxy, bevorzugt C₁ bis C₃₂-Alkyl oder Alkoxy, besonders bevorzugt C₁ bis C₂₈-Alkyl oder Alkoxy, ganz besonders bevorzugt C₁ bis C₂₆-Alkyl oder Alkoxy und insbesondere ganz besonders bevorzugt C₁ bis C₂₄-Alkyl oder Alkoxy, gegebenenfalls substituiertes C₆ bis C₁₄-Aryl oder Aryloxy bevorzugt C₆ bis C₁₀- Aryl oder Aryloxy oder C₇ bis C₃₀-Aralkyl, besonders bevorzugt C₇ bis C₂₄-Aralkyl stehen,
n und m unabhängig voneinander für eine ganze Zahl zwischen 0 und 5, bevorzugt für 1 bis 3, besonders bevorzugt für 1 bis 2 und ganz besonders bevorzugt für 1 stehen, wobei es sich auch um Isomerengemische handeln kann.

Bevorzugt sind auch Verbindungen der Formel (1) in denen mindestens einer der Reste R₃ und R₄ unabhängig von einander aus den oben definierten Alkylsubstituenten ausgewählt ist.

Bevorzugt sind auch Verbindungen der Formel (1) in denen mindestens einer der Reste R₃ und R₄ unabhängig von einander aus den oben definierten Alkyloxysubstituenten ausgewählt ist.

Bevorzugt sind auch Verbindungen der Formel (1) in denen mindestens einer der Reste R₃ und R₄ unabhängig von einander aus den oben definierten Arylsubstituenten ausgewählt ist.

Bevorzugt sind auch Verbindungen der Formel (1) in denen mindestens einer der Reste R₃ und R₄ unabhängig von einander aus den oben definierten Aryloxysubstituenten ausgewählt ist.

Bevorzugt sind auch Verbindungen der Formel (1) in denen mindestens einer der Reste R₃ und R₄ unabhängig von einander aus den oben definierten Aralkylsubstituenten ausgewählt ist.

Ganz besonders bevorzugt werden die aromatischen Formale durch die allgemeine Formel (2) beschrieben 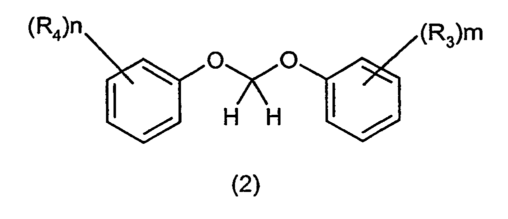 worin
R₃, R₄, m und n die oben genannten Bedeutungen haben und wobei es sich auch um Isomerengemische handeln kann.

Ganz besonders bevorzugt sind weiterhin die Verbindungen der Formeln (1) und (2) in denen R₃ und R₄ die gleiche Bedeutung haben.

Die vorliegende Erfindung betrifft weiterhin ein Verfahren zur Herstellung von Formalen der Formeln (1) und (2) dadurch gekennzeichnet, dass monofunktionelle Phenole der Formel 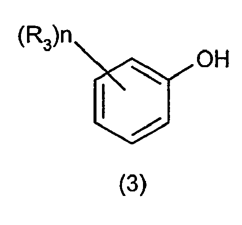 worin
R₃ und n die oben genannten Bedeutungen haben,
in einer homogenen Lösung aus Methylenchlorid oder α,α-Dichlortoluol und einem geeigneten hochsiedenen Lösungsmittel wie beispielsweise N-Methylpyrrolidon (NMP), Dimethylformamid (DMF), N-Methylcaprolactam (NMC), Chlorbenzol, Dichlorbenzol oder Tetrahydrofuran (THF) in Gegenwart einer Base, bevorzugt Natriumhydroxid (NaOH) oder Kaliumhydroxid (KOH) und ganz besonders NaOH, bevorzugt bei Temperaturen zwischen 30 und 80°C, besonders bevorzugt zwischen 50 und 80°C und ganz besonders bevorzugt zwischen 60 und 80°C zur Reaktion gebracht werden. Bevorzugte hochsiedende Lösungsmittel sind NMP, DMF und NMC, besonders bevorzugt NMP und NMC und ganz besonders bevorzugt NMP.

Die Phenole der Formel (3) sind bekannt oder können nach literaturbekannten Verfahren, beispielsweise durch Alkylierung nach Friedel Crafts, hergestellt werden (Organikum, Organischchemisches Grundpraktikum, Korrigierter Nachdruck der 20. Auflage, Wiley-VCH, Weinheim, S. 355, 1999). Sehr viele Phenole sind auch käuflich erhältlich (Liferanten z.B. Aldrich, Fluka, Acros usw.). Die Phenole der Formel (3) können allein als Isomerengemisch oder als Mischung eingesetzt werden.

Die erfindungsgemäßen Polymermischungen enthalten die aromatischen Formale im Allgemeinen zu einem Anteil von 10 - 60000 ppm, bevorzugt 10 - 50000 ppm, besonders bevorzugt 20 - 40000 ppm, ganz besonders bevorzugt zwischen 50 und 30000 ppm.

Bevorzugt, besonders bevorzugt oder ganz besonders bevorzugt sind Ausführungsformen, welche von den unter bevorzugt, besonders bevorzugt oder ganz besonders bevorzugt genannten Parametern, Verbindungen, Definitionen und Erläuterungen Gebrauch machen.

Die in der Beschreibung aufgeführten allgemeinen oder in Vorzugsbereichen aufgeführten Definitionen, Parameter, Verbindungen und Erläuterungen können jedoch auch untereinander, also zwischen den jeweiligen Bereichen und Vorzugsbereichen beliebig kombiniert werden.

Weiterhin sind Gegenstand der Erfindung die Verwendung solcher Formmassen zur Herstellung von optischen Datenträgern, wie z.B. Compact Disks, Video Disks, Digital Versatile Disks und weitere ein- oder mehrfach schreib- wie löschbare optische Datenträger, sowie die aus den Polymermischungen herstellbaren optischen Datenträger selbst.

Selbstverständlich kann das Polymergemisch auch für andere tradionelle Polycarbonat-Anwendungen, auch in solchen die ein Polycarbonat mit einem höheren Molekulargewicht benutzen, benutzt werden. Die Anwendungen können transparent oder undurchsichtig sein wie zum Beispiele: Lebensmittel- und Getränkeverpackungen, optische Linsen und Prismen, Linsen für Beleuchtungszwecke, Autoscheinwerferlinsen, Verglasungen für Bau- und Kraftfahrzeuge, Verscheibungen anderer Art wie für Gewächshäuser, sogenannte Stegdoppelplatten oder Hohlkammerplatten. Andere Beispiele der Anwendungen sind Profile, Folien, Gehäuseteile jeder Art, z.B. für Medizinische Geräte, Haushaltsgeräte wie Saftpressen, Kaffeemaschinen, Mixer; für Büromaschinen wie Computer, Monitore, Drucker, Kopierer; für Platten, Rohre, Elektroinstallationskanäle, Fenster, Türen und Profile für den Bausektor, Innenausbau und Außenanwendungen; auf dem Gebiet der Elektrotechnik z.B. für Schalter und Stecker. Ferner können die erfindungsgemäßen Formkörper für Innenausbau- und Bauteile von Schienenfahrzeugen, Schiffen, Flugzeugen, Bussen und anderen Kraftfahrzeugen sowie für Kfz-Karosserieteile verwendet werden.

Thermoplastische Formmassen im Sinne der vorliegenden Erfindung enthalten überwiegend aromatische Polycarbonate. Unter Polycarbonate sind sowohl Homopolycarbonate als auch Copolycarbonate zu verstehen; die Polycarbonate können in bekannter Weise linear oder verzweigt sein. Sie weisen ein durch Gelpermeationschromatographie bestimmtes Gewichtsmittel des Molekulargewichts von 5.000 bis 80.000, vorzugsweise 10.000 bis 40.000 auf. Besonders bevorzugt liegt das Molekulargewicht zwischen 15 000 und 35 000, insbesondere 15 000 und 22 000.

Die Herstellung dieser Polycarbonate erfolgt in bekannter Weise aus Diphenolen, Kohlensäurederivaten, gegebenenfalls Kettenabbrechern und gegebenenfalls Verzweigern.

Einzelheiten der Herstellung von Polycarbonaten sind in vielen Patentschriften seit etwa 40 Jahren niedergelegt. Beispielhaft sei hier nur auf Schnell, "Chemistry and Physics of Polycarbonates", Polymer Reviews, Volume 9, Interscience Publishers, New York, London, Sydney 1964, auf D. Freitag, U. Grigo, P.R. Müller, H. Nouvertne, BAYER AG, "Polycarbonates" in Encyclopedia of Polymer Science and Engineering, Volume 11, Second Edition, 1988, Seiten 648-718 und schließlich auf Dres. U. Grigo, K. Kirchner und P.R. Müller "Polycarbonate" in Becker/Braun, Kunststoff-Handbuch, Band 3/1, Polycarbonate, Polyacetale, Polyester, Celluloseester, Carl Hanser Verlag München, Wien 1992, Seiten 117-299 verwiesen. ,

Für die Herstellung der Polycarbonate geeignete Diphenole sind beispielsweise Hydrochinon, Resorcin, Dihydroxydiphenyle, Bis-(hydroxyphenyl)-alkane, Bis(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole, sowie deren kernalkylierte und kernhalogenierte Verbindungen.

Bevorzugte Diphenole sind 4,4'-Dihydroxydiphenyl, 2,2-Bis-(4-hydroxyphenyl)-propan, 2,4-Bis-(4-hydroxyphenyl)-2-methylbutan, 1, 1-Bis-(4-hydroxyphenyl)-p-diisopropylbenzol, 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,4-Bis-[2-(4-hydroxyphenyl)-2-propyl]benzol, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-3,3,5-trimethylcyclohexan und 1,3-Bis-[2-(4-hydroxyphenyl)-2-propyl]benzol.

Besonders bevorzugte Diphenole sind 2,2-Bis-(4-hydroxyphenyl)-propan (BPA), 2,2-Bis-(3,5-dimethyl-4-hydroxyphenyl)-propan, 2,2-Bis-(3,5-dichlor-4-hydroxyphenyl)-propan, 2,2-Bis-(3,5-dibrom-4-hydroxyphenyl)-propan, 1,3-Bis-[2-(4-hydroxyphenyl)-2-propyl]benzol (BPM), 1,1-Bis-(4-hydroxyphenyl)-cyclohexan und 1,1-Bis-(4-hydroxyphenyl)-3,3,5-trimethylcyclohexan (TMC).

Diese und weitere geeignete Diphenole sind z.B. in den US 3 028 635, 2 999 835, 3 148 172, 2 991 273, 3 271 367, 4 982 014 und 2 999 846, in den DE-A 1 570 703, 2 063 050, 2 036 052, 2 211 956 und 3 832 396, der französischen Patentschrift 1 561 518, in der Monographie "H. Schnell, Chemistry and Physics of Polycarbonates, Interscience Publishers, New York 1964" sowie in den JP-A 62039/1986, 62040/1986 und 105550/1986 beschrieben.

Im Falle der Homopolycarbonate ist nur ein Diphenol eingesetzt, im Falle der Copolycarbonate sind mehrere Diphenole eingesetzt.

Bevorzugt werden Formmassen verwendet, die mindestens ein Polycarbonat mit Diolbausteinen aus BPA und/oder Trimethylcyclohexyl-bisphenol (TMC) enthalten, bevorzugt ausgewählt aus der Gruppe der Homopolymere des BPA, der Copolymere des BPA mit TMC oder der Copolymere mit 5 bis 60 Gew.-% TMC.

Geeignete Kohlensäurederivate sind beispielsweise Phosgen oder Diphenylcarbonat.

Geeignete Kettenabbrecher sind sowohl Monophenole als auch Monocarbonsäuren. Geeignete Monophenole sind Phenol selbst, Alkylphenole wie Kresole, p-tert.-Butylphenol, p-n-Octylphenol, p-isoOctylphenol, p-n-Nonylphenol und p-iso-Nonylphenol, p-Cumylphenol, Halogenphenole wie p-Chlorphenol, 2,4-Dichlorphenol, p-Bromphenol Amylphenol und 2,4,6-Tribromphenol sowie deren Mischungen.

Bevorzugte Kettenabbrecher sind die Phenole der Formel (I) 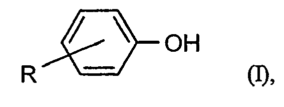 worin R Wasserstoff, tert.-Butyl oder ein verzweigter oder unverzweigter C₈- und/oder C₉-Alkylrest ist. Aber auch p-Cumylphenol kann bevorzugt verwendet werden. Im Falle des Umesterungsverfahrens resultiert der Kettenabbrecher aus dem eingesetzten Diarylcarbonat.

Die Menge an einzusetzendem Kettenabbrecher, bevorzugt im Phasengrenzflächenverfahren, beträgt 0,1 Mol-% bis 5 Mol-%, bezogen auf Mole an jeweils eingesetzten Diphenolen. Die Zugabe der Kettenabbrecher kann vor, während oder nach der Phosgenierung erfolgen.

Geeignete Verzweiger sind die in der Polycarbonatchemie bekannten tri- oder mehr als trifunktionellen Verbindungen, insbesondere solche mit drei oder mehr als drei phenolischen OH-Gruppen.

Geeignete Verzweiger sind beispielsweise Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1 -Tri-(4-hydroxyphenyl)-ethan, Tri-(4-hydroxyphenyl)-phenylmethan, 2,2-Bis-[4,4-bis-(4-hydroxyphenyl)-cyclohexyl]-propan, 2,4-Bis-(4-hydroxyphenyl-isopropyl)-phenol, 2,6-Bis-(2-hydroxy-5'-methyl-benzyl)-4-methylphenol, 2-(4-Hydroxyphenyl)-2-(2,4-dihydroxyphenyl)-propan, Hexa-(4-(4-hydroxyphenyl-isopropyl)-phenyl)-orthoterephthalsäureester, Tetra-(4-hydroxyphenyl)-methan, Tetra-(4-(4 hydroxyphenyl-isopropyl)-phenoxy)-methan und 1,4-Bis-(4',4"-dihydroxytriphenyl)-methyl)-benzol sowie 2,4-Dihydroxybenzoesäure, Trimesinsäure, Cyanurchlorid und für einige Anwendungen sogar bevorzugt 3,3-Bis-(3-methyl-4-hydroxyphenyl)-2-oxo-2,3-dihydroindol.

Die Menge der gegebenenfalls einzusetzenden Verzweiger beträgt 0,01 Mol-% bis 2 Mol-%, bezogen wiederum auf Mole an jeweils eingesetzten Diphenolen.

Die Verzweiger können im Phasengrenzflächenverfahren entweder mit den Diphenolen und den Kettenabbrechern in der wässrig alkalischen Phase vorgelegt werden, oder in einem organischen Lösungsmittel gelöst zugegeben werden. Im Falle des Umesterungsverfahrens können die Verzweiger zusammen mit den Diphenolen eingesetzt werden.

Alle diese Maßnahmen zur Herstellung der thermoplastischen Polycarbonate sind dem Fachmann geläufig.

Den erfindungsgemäß thermoplastischen Polymermischungen können weiterhin für Polycarbonate übliche Zusätze in den bekannten Mengen enthalten, wie beispielhaft und vorzugsweise Stabilisatoren gegen UV-Strahlung, Flammschutzmittel, Farbstoffe, Füllstoffe, Schaummittel, optische Aufheller und Antistatika. Bei optischen Anwendungen werden bevorzugt solche Komponenten genommen, die die Transparenz des Materials nicht negativ beeinflussen.

Diese Substanzen sind in vielen Veröffentlichungen zu finden wie etwa in Additives for Plastics Handbook, John Murphy, 1999 und im Handel erhältlich.
1. Geeignete Antioxidantien sind beispielsweise:
   1.1. Alkylierte Monophenole, zum Beispiel 2,6-Di-tert-butyl-4-methylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Dicyclopentyl-4-methylphenol, 2-(α-Methylcyclohexyl)-4,6-dimethylphenol, 2,6-Dioctadecyl-4-methylphenol, 2,4,6-Tricyclohexylphenol, 2,6-Di-tert-butyl-4-methoxymethylphenol, Nonylphenole, die in der Seitenkette linear oder verzweigt sind, zum Beispiel, 2,6-Di-nonyl-4-methylphenol, 2,4-Dimethyl-6-(1'-methylundec-1'-yl)phenol, 2,4-Dimethyl-6-(1'-methylheptadec-1'-yl)phenol, 2,4-Dimethyl-6-(1'-methyltridec-1'-yl)phenol.
   1.2. Alkylthiomethylphenole, zum Beispiel 2,4-Dioctylthiomethyl-6-tert-butylphenol, 2,4-Dioctylthiomethyl-6-methylphenol, 2,4-Dioctylthiomethyl-6-ethylphenol, 2,6-Didodecylthiomethyl-4-nonylphenol.
   1.3. Hydrochinone und alkylierte Hydrochinone, zum Beispiel 2,6-Di-tert-butyl-4-methoxyphenol, 2,5-Di-tert-butylhydrochinon, 2,5-Di-tert-amylhydrochinon, 2,6-Diphenyl-4-octadecyloxyphenol, 2,6-Di-tert-butylhydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis(3,5-di-tert-butyl-4-hydroxyphenyl)adipat.
   1.4. Tocopherole, zum Beispiel α-Tocopherol, β-Tocopherol, γ-Tocopherol, δ-Tocopherol und Gemische davon (Vitamin E).
   1.5. Hydroxylierte Thiodiphenylether, zum Beispiel 2,2'-Thiobis(6-tert-butyl-4-methylphenol), 2,2'-Thiobis(4-octylphenol), 4,4'-Thiobis(6-tert-butyl-3-methyl-phenol), 4,4'-Thiobis(6-tert-butyl-2-methylphenol), 4,4'-Thiobis(3,6-di-sec-amylphe-nol), 4,4'-Bis(2,6-dimethyl-4-hydroxyphenyl)-disulfid.
   1.6. Alkylidenbisphenole, zum Beispiel 2,2'-Methylenbis(6-tert-butyl-4-methylphenol), 2,2'-Methylenbis(6-tert-butyl-4-ethylphenol), 2,2'-Methylenbis[4-methyl-6-(α-methylcyclohexyl)-phenol], 2,2'-Methylenbis(4-methyl-6-cyclohexylphe-nol), 2,2'-Methylenbis(6-nonyl-4-methylphenol), 2,2'-Methylenbis(4,6-di-tert-butyl-phenol), 2,2'-Ethylidenbis(4,6-di-tert-butylphenol), 2,2'-Ethylidenbis(6-tert-butyl-4-isobutylphenol), 2,2'-Methylenbis[6-(α-methylbenzyl)-4-nonylphenol], 2,2'-Methylenbis[6-(α,α-dimethylbenzyl)-4-nonylphenol], 4,4'-Methylenbis(2,6-di-tert-butylphenol), 4,4'-Methylenbis(6-tert-butyl-2-methylphenol), 1,1-Bis(S-tert-butyl-4-hydroxy-2-methylphenyl)butan, 2,6-Bis(3-tert-butyl-5-methyl-2-hydroxybenzyl)-4-methylphenol, 1,1,3-Tris(5-tert-butyl-4-hydroxy-2-methylphenyl)butan, 1,1-Bis(5-tert-butyl-4-hydroxy-2-methylphenyl)-3-n-dodecylmercaptobutan, Ethylenglycolbis[3,3-bis(3'-tert-butyl-4'-hydroxyphenyl)butyrat], Bis(3-tert-butyl-4-hydroxy-5-methyl-phenyl)dicyclopentadien, Bis[2-(3'-tert-butyl-2'-hydroxy-5'-methylbenzyl)-6-tert-butyl-4-methylphenyl]terephthalat, 1,1-Bis(3,5-dimethyl-2-hydroxyphenyl)butan, 2,2-Bis(3,5-di-tert-butyl-4-hydroxyphenyl)propan, 2,2-Bis(5-tert-butyl-4-hydroxy-2-methylphenyl)-4-n-dodecylmercaptobutan, 1,1,5,5-Tetra-(5-tert-butyl-4-hydroxy-2-methylphenyl)pentan.
   1.7. O-, N- und S-Benzylverbindungen, zum Beispiel 3,5,3',5'-Tetra-tert-butyl-4,4'-dihydroxydibenzylether, Octadecyl-4-hydroxy-3,5-dimethylbenzylmercaptoacetat, Tridecyl-4-hydroxy-3,5-di-tert-butylbenzylmercaptoacetat, Tris(3,5-di-tert-butyl-4-hydroxybenzyl)amin, Bis(4-tert-butyl-3-hydroxy-2,6-dimethylbenzyl)dithioterephthalat, Bis(3,5-di-tert-butyl-4-hydroxybenzyl)sulfid, Isooctyl-3,5-di-tert-butyl-4-hydroxybenzylmercaptoacetat.
   1.8. Hydroxybenzylierte Malonate, zum Beispiel Dioctadecyl-2,2-bis(3,5-di-tert-butyl-2-hydroxybenzyl)malonat, Dioctadecyl-2-(3-tert-butyl-4-hydroxy-5-methyl-benzyl)malonat, Didodecylmercaptoethyl-2,2-bis(3,5-di-tert-butyl-4-hydroxybenzyl) malonat, Bis[4-(1,1,3,3-tetramethylbutyl)-phenyl]-2,2-bis(3,5-di-tert-butyl-4-hydroxy-benzyl)malonat.
   1.9. Aromatische Hydroxybenzylverbindungen, zum Beispiel 1,3,5-Tris-(3,5-di-tert-butyl-4-hydroxybenzyl)-2,4,6-trimethylbenzol, 1,4-Bis(3,5-di-tert-butyl-4-hydroxy-benzyl)-2,3,5,6-tetramethylbenzol, 2,4,6-Tris(3,5-di-tert-butyl-4-hydroxybenzyl)phenol.
   1.10. Triazinverbindungen, zum Beispiel 2,4-Bis(octylmercapto)-6-(3,5-di-tert-butyl-4-hydroxyanilino)-1,3,5-triazin, 2-Octylmercapto-4,6-bis(3,5-di-tert-butyl-4-hydroxy-anilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris-(3,5-di-tert-butyl-4-hydroxybenzyl)isocyanurat, 1,3,5-Tris(4-tert-butyl-3-hydroxy-2,6-dimethylbenzyl)isocyanurat, 2,4,6-Tris(3,5-di-tert-butyl-4-hydroxyphenylethyl)-1,3,5-triazin, 1,3,5-Tris(3,5-di-tert-butyl-4-hydroxyphenylpropioyl)-hexahydro-1,3,5-triazin, 1,3,5-Tris(3,5-dicyclohexyl-4-hydroxybenzyl)isocyanurat.
   1.11. Acylaminophenole, zum Beispiel 4-Hydroxylauranilid, 4-Hydroxytearanilid, Octyl-N-(3,5-di-tert-butyl-4-hydroxyphenyl)carbamat.
   1.12. Ester von β-(3,5-Di-tert-butyl-4-hydroxyphenyl)propionsäure mit ein- oder mehrwertigen Alkoholen, z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)isocyanurat, N,N'-Bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan, ganz besonder geeignet und bevorzugt ist dabei der Ester mit Octadecanol (IRGANOX 1076 ® der Ciba Spec.)
   1.13. Ester von β-(5-tert-Butyl-4-hydroxy-3-methyphenyl)propionsäure mit ein- oder mehrwertigen Alkoholen, z.B. mit Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)isocyanurat, N,N'-Bis-(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan.
   1.14. Ester von β-(3,5-Dicyclohexyl-4-hydroxyphenyl)propionsäure mit ein- oder mehrwertigen Alkoholen, z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl) isocyanurat, N,N'-Bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabi-cyclo[2.2.2]octan.
   1.15. Ester von 3.5-Di-tert-butyl-4-hydroxyphenylessigsäure mit ein- oder mehrwertigen Alkoholen, z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)isocyanurat, N,N'-Bis(hydroxyethyl)oxamid, 3-Thiaundecanol, 3-Thiapentadecanol, Trimethylhexandiol, Trimethylolpropan, 4-Hydroxymethyl-1-phospha-2,6,7-trioxabicyclo[2.2.2]octan.
   1.16. Amide of β-(3,5-Di-tert-butyl-4-hydroxyphenyl)propionsäure, z.B. N,N'-Bis(3,5-di-tert-butyl-4-hydroxyphenylpropionyl)hexamethylendiamid, N,N'-Bis(3,5-di-tert-butyl-4-hydroxyphenylpropionyl)trimethylendiamid, N,N'-Bis(3,5-di-tert-butyl-4-hydroxyphenylpropionyl)hydrazid, N,N'-Bis[2-(3-[3,5-di-tert-butyl-4-hydroxyphenyl]-propionyloxy)ethyl]oxamid (Naugard® XL-1 von Uniroyal).
   1.17. Ascorbinsäure (Vitamin C)
   1.18. Aminische Antioxidantien, zum Beispiel NN'-Diisopropyl-p-phenylendiamin, N,N'-Di-sec-butyl-p-phenylendiamin, N,N'-Bis(1,4-dimethylpentyl)-p-phenylendiamin, N,N'-Bis(1-ethyl-3-methylpentyl)-p-phenylendiamin, N,N'-Bis(1-methylheptyl)-p-phenylendiamin, N,N'-Dicyclohexyl-p-phenylendiamin, N,N'-Diphenyl-p-phenylen-diamin, N,N'-Bis(2-naphthyl)-p-phenylendiamin, N-Isopropyl-N'-phenyl-p-phenylen-diamin, N-(1,3-Dimethylbutyl)-N'-phenyl-p-phenylendiamin, N-(1-Methylheptyl)-N'-phenyl-p-phenylendiamin, N-Cyclohexyl-N'-phenyl-p-phenylendiamin, 4-(p-Toluolsulfamoyl)diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-(4-tert-Octylphenyl)-1-naphthylamin, N-Phenyl-2-naphthylamin, octyliertes Diphenylamin, zum Beispiel p,p'-Di-tert-octyl-diphenylamin, 4-n-Butylaminophenol, 4-Butyrylaminophenol, 4-Nonanoyl-aminophenol, 4-Dodecanoylaminophenol, 4-Octadecanoylaminophenol, Bis(4-methoxyphenyl)amin, 2,6-Di-tert-butyl-4-dimethylaminomethylphenol, 2,4'-Diaminodiphenylmethan, 4,4'-Diaminodiphenylmethan, N,N,N',N'-Tetramethyl-4,4'-diaminodiphenylmethan, 1,2-Bis[(2-methylphenyl)amino]ethan, 1,2-Bis(phenyl-amino)propan, (o-Tolyl)biguanid, Bis[4-(1',3'-dimethylbutyl)phenyl]amin, tert-octyliertes N-Phenyl-1-naphthylamin, ein Gemisch von mono- und dialkylierten tert-Butyl/tert-Octyldiphenylaminen, ein Gemisch von mono- und dialkylierten Nonyldiphenylaminen, ein Gemisch von mono- und dialkylierten Dodecyldiphenylaminen, ein Gemisch von mono- und dialkylierten Isopropyl/Isohexyldiphenylaminen, ein Gemisch von mono- und dialkylierten tert-Butyldiphenylaminen, 2,3-Dihydro-3,3-dimethyl-4H-1,4 benzothiazin, Phenothiazin, ein Gemisch von mono- und dialkylierten tert-Butyl/tert-Octylphenothiazinen, ein Gemisch von mono- und dialkylierten tert-Octylphenothiazinen, N-Allylphenothiazin, N,N,N',N'-Tetraphenyl-1,4-diaminobut-2-en, N,N-bis(2,2,6,6-tetramethylpiperid-4-ylhexamethylendiamin, Bis(2,2,6,6-tetramethylpiperid-4-yl)sebacat, 2,2,6,6-Tetramethylpiperidin-4-on, 2,2,6,6-Tetra-methylpiperidin-4-ol. Es können einzelne dieser Verbindungen oder Gemische derselben eingesetzt werden.
   1.19 Geeignete Thiosynergisten sind zum Beispiel Dilaurylthiodipropionat und/oder Distearylthiodipropionat.
2. UV-Absorber und Lichtstabilisatoren können in den erfindungsgemäßen Zusammensetzungen in Mengen von 0,1 bis 15 Gew.-%, vorzugsweise 3 bis 8 Gew.-%, bezogen auf die Masse der Zusammensetzung, eingesetzt werden. Geeignete UV-Absorber und Lichtstabilisatoren sind beispielsweise:
   2.1. 2-(2'-Hydroxyphenyl)benzotriazole, um Beispiel 2-(2'-Hydroxy-5'-methylphenyl)benzotriazol, 2-(3',5'-Di-tert butyl-2'-hydroxyphenyl)benzotriazol, 2-(5'-tert-Butyl-2'-hydroxyphenylbenzotriazol, 2-(2'-Hydroxy-5'-(1,1,3,3-tetramethylbutyl) phenyl)benzo-triazol, 2-(3',5'-Di-tert-butyl-2'-hydroxyphenyl)-5-chlorbenzo-triazol, 2-(3'-tert-Butyl-2'-hydroxy-5'-methylphenyl)-5-chlorbenzotriazol, 2-(3'-sec-Butyl-5'-tert-butyl-2'-hydroxyphenyl)benzotriazol, 2-(2'-Hydroxy-4'-octyloxyphenyl)benzotriazol, 2-(3',5'-Di-tert-amyl-2'-hydroxyphenyl)benzotriazol, 2-(3',5'-Bis(α,α-dimethylbenzyl)-2'-hydroxyphenyl)benzotriazol, 2-(3'-tert-Butyl-2'-hydroxy-5'-(2-octyloxycarbonylethyl)phenyl)-5-chlorbenzotriazol, 2-(3'-tert-Butyl-5'-[2-(2-ethyl-hexyloxy)carbonyl-ethyl]-2'-hydroxyphenyl)-5-chlorbenzotriazol, 2-(3'-tert-Butyl-2'-hydroxy-5'-(2-meth-oxycarbonylethyl)-phenyl)-5-chlorbenzotriazol, 2-(3'-tert-Butyl-2'-hydroxy-5'-(2-methoxycarbonylethyl)-phenyl)benzotriazol, 2-(3'-tert-Butyl-2'-hydroxy-5'-(2-octyl-oxycarbonylethyl)phenyl)benzotriazol, 2-(3'-tert-Butyl-5'-[2-(2-ethylhexyloxy)carbonylethyl]-2'-hydroxyphenyl)benzotriazol, 2-(3'-Dodecyl-2'-hydroxy-5'-methylphenyl)benzotriazol, 2-(3'-tert-Butyl-2'-hydroxy-5'-(2-isooctyl-oxycarbonylethyl)phenylbenzotriazol, 2,2'-Methylenbis[4-(1,1,3,3-tetramethylbutyl)-6-benzotriazol-2-ylphenol]; das Umesterungsprodukt von 2-[3'-tert-Butyl-5'-(2-methoxycarbonylethyl)-2'-hydroxyphenyl]-2H-benzotriazol mit Polyethylenglycol 300; [R-CH₂CH₂-COO-CH₂CH₂]₂, wobei R = 3'-tert-Butyl-4'-hydroxy-5'-2H-benzotriazol-2-ylphenyl, 2-[2'-Hydroxy-3'-(α,α-dimethylbenzyl)-5'-(1,1,3,3-tetra-methylbutyl)phenyl]benzotriazol, 2-[2'-Hydroxy-3'-(1,1,3,3-tetramethylbutyl)-5'-(α,α -dimethylbenzyl)phenyl]benzotriazol.
   2.2. 2-Hydroxybenzophenone, zum Beispiel die 4-Hydroxy-, 4-Methoxy-, 4-Octyloxy-, 4-Decyloxy-, 4-Dodecyloxy-, 4-Benzyloxy-, 4,2',4'-Trihydroxy- und 2'-Hydroxy-4,4'-dimethoxy-Derivate.
   2.3. Ester von substituierten und unsubstituierten Benzoesäuren, wie zum Beispiel 4-tert-Butylphenylsalicylat, Phenylsalicylat, Octylphenylsalicylat, Bibenzoylresorcin, Bis(4-tert-butylbenzoyl)resorcin, Benzoylresorcin, 2,4-Di-tert-butylphenyl-3,5-di-tert-butyl-4-hydroxybenzoat, Hexadecyl-3,5-di-tert-butyl-4-hydroxybenzoat, Octadecyl-3,5-di-tert-butyl-4-hydroxybenzoat, 2-Methyl-4,6-di-tert-butylphenyl-3,5-di-tert-butyl-4-hydroxybenzoat.
   2.4. Acrylate, zum Beispiel Ethyl-α-cyan-β,β-diphenylacrylat, Isooctyl-α-cyan-β,β-diphenylacrylat, Methyl-α-carbomethoxycinnamat, Methyl-α-cyan-β-methyl-p-methoxycinnamat, Butyl-α-cyan-β-methyl-p-methoxycinnamat, Methyl-α-carbomethoxy-p-methoxycinnamat und N-(β-Carbomethoxy-β-cyanvinyl)-2-methylindolin.
   2.5. Nickelverbindungen, zum Beispiel Nickelkomplexe von 2,2'-Thiobis[4-(1,1,3,3-tetramethylbutyl)phenol], wie der 1:1- oder 1:2-Komplex, mit oder ohne zusätzliche Liganden, wie n-Butylamin, Triethanolamin oder N-Cyclohexyldiethanolamin, Nickeldibutyldithiocarbamat, Nickelsalze der Monoalkylester, z.B. des Methyl- oder Ethylesters, von 4-Hydroxy-3,5-di-tert-butylbenzylphosphonsäure, Nickelkomplexe von Ketoximen, z.B. von 2-Hydroxy-4-methylphenylundecylketoxim, Nickelkomplexe von 1-Phenyl-4-lauroyl-5-hydroxypyrazol, mit oder ohne zusätzliche Liganden.
   2.6. Sterisch gehinderte Amine, zum Beispiel Bis(2,2,6,6-tetramethyl-4-piperidyl)sebacat, Bis(2,2,6,6-tetramethyl-4-piperidyl)succinat, Bis(1,2,2,6,6-pentamethyl-4-piperidyl)sebacat, Bis(1-octyloxy-2,2,6,6-tetramethyl-4-piperidyl)sebacat, Bis(1,2,2,6,6-pentamethyl-4-piperidyl), n-Butyl-3,5-di-tert-butyl-4-hydroxy-benzylmalonat, das Kondensat von 1-(2-Hydroxyethyl)-2,2,6,6-tetramethyl-4-hydroxypiperidin und Bernsteinsäure, lineare oder cyclische Kondensate von N,N'-Bis(2,2,6,6-tetramethyl-4-piperidyl)hexamethylendiamin und 4-tert-Octylamino-2,6-dichlor-1,3,5-triazin, Tris(2,2,6,6-tetramethyl-4-piperidyl)nitrilotriacetat, Tetrakis(2,2,6,6-tetramethyl-4-piperidyl)-1,2,3,4-butantetracarboxylat, 1,1'-(1,2-Ethandiyl) bis(3,3,5,5-tetramethylpiperazinon), 4-Benzoyl-2,2,6,6-tetramethylpiperidin, 4-Stearyloxy-2,2,6,6-tetramethylpiperidin, Bis(1,2,2,6,6-pentamethylpiperidyl)-2-n-butyl-2-(2-hydroxy-3,5-di-tert-butylbenzyl)malonat, 3-n-Octyl-7,7,9,9-tetramethyl-1,3,8-triazaspiro[4.5]decan-2,4-dion, Bis(1-octyloxy-2,2,6,6-tetramethyl-piperidyl) sebacat, Bis(1-octyloxy-2,2,6,6-tetramethylpiperidyl)succinat, lineare oder cyclische Kondensate von N,N'-Bis(2,2,6,6-tetramethyl-4-piperidyl)hexamethylendiamin und 4-Morpholino-2,6-dichlor-1,3,5-triazin, das Kondensat von 2-Chlor-4,6-bis(4-n-butylamino-2,2,6,6-tetramethylpiperidyl)-1,3,5-triazin und 1,2-Bis(3-aminopropyl-amino)ethan, das Kondensat von 2-Chlor-4,6-bis(4-n-butylamino-1,2,2,6,6-penta-methylpiperidyl)-1,3,5-triazin und 1,2-Bis(3-aminopropylamino)ethan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4 piperidyl)pyrrolidin-2,5-dion, ein Gemisch von 4-Hexadecyloxy- und 4-Stearyloxy-2,2,6,6-tetramethylpiperidin, ein Kondensationsprodukt von N,N'-Bis(2,2,6,6-tetramethyl-4-piperidyl)hexamethylendiamin und 4-Cyclohexylamino-2,6-dichloro-1,3,5-triazin, ein Kondensationsprodukt von 1,2-Bis-(3-aminopropylamino)ethan und 2,4,6-Trichlor-1,3,5-triazin sowie 4-Butylamino-2,2,6,6-tetramethylpiperidin (CAS Reg. No. [136504-96-6]); N-(2,2,6,6-Tetramethyl-4-piperidyl)-n-dodecylsuccinimid, N-(1,2,2,6,6-Pentamethyl-4-piperidyl)-n-dodecyl-succinimid, 2-Undecyl-7,7,9,9-tetramethyl-1-oxa-3,8-diaza-4-oxospiro[4.5]decan, ein Reaktionsprodukt von 7,7,9,9-Tetramethyl-2-cycloundecyl-1-oxa-3,8-diaza-4-oxospiro[4.5]decan und Epichlorhydrin, 1,1-Bis(1,2,2,6,6-pentamethyl-4-piperidyl-oxycarbonyl)-2-(4-methoxyphenyl)ethen, N,N'-Bis(formyl)-N,N'-bis(2,2,6,6-tetra-methyl-4-piperidyl)hexamethylendiamin, Diester von 4-Methoxymethylenmalonsäure mit 1,2,2,6,6-Pentamethyl-4-hydroxypiperidin, Poly[methylpropyl-3-oxy-4-(2,2,6,6-tetramethyl-4-piperidyl)]siloxan, Reaktionsprodukt von Maleinsäureanhydrid-α-Olefin-Copolymer mit 2,2,6,6-Tetramethyl-4-aminopiperidin oder 1,2,2,6,6-Pentamethyl-4-aminopiperidin.
   2.7. Oxamide, zum Beispiel 4,4'-Dioctyloxyoxanilid, 2,2'-Diethoxyoxanilid, 2,2'-Dioctyloxy-5,5'-di-tert-butoxanilid, 2,2'-Didodecyloxy-5,5'-di-tert-butoxanilid, 2-Ethoxy-2'-ethyloxanilid, N,N'-Bis(3-dimethylaminopropyl)oxamid, 2-Ethoxy-5-tert-butyl-2'-ethoxanilid und dessen Gemisch mit 2-Ethoxy-2'-ethyl-5,4'-di-tert-butoxanilid, Gemische von o- und p-Methoxy-disubstituierten Oxaniliden und Gemische von o- und p-Ethoxy-disubstituierten Oxaniliden.
   2.8. 2-(2-Hydroxyphenyl)-1,3,5-triazine, zum Beispiel 2,4,6-Tris(2-hydroxy-4-octyloxyphenyl)-1,3,5-triazin, 2-(2-Hydroxy-4-octyloxyphenyl)-4,6-bis(2,4-dimethyl-phenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxypropoxy)phenyl]-4,6-bis(2,4-dimethyl)-1,3,5-triazin, 2-[2-Hydroxy-4-(2-hydroxy-3-octyloxypropyloxy)phenyl]-4,6-bis(2,4-dimethyl)-1,3,5-triazin, 2-[4-(Dodecyloxy/Tridecyloxy-2-hydroxypropoxy)-2-hydroxyphenyl]-4,6-bis(2,4-dimethylphenyl)-1,3,5-triazin, 2-[2-Hydroxy-4-(2-hydroxy-3-dodecyloxypropoxy)phenyl]-4,6-bis(2,4-dimethylphenyl)-1,3,5-triazin, 2-(2-Hydroxy-4-hexyloxy)phenyl-4,6-diphenyl-1,3,5-triazin, 2-(2-Hydroxy-4-methoxy-phenyl)-4,6-diphenyl-1,3,5-triazin, 2,4,6-Tris[2-hydroxy-4-(3-butoxy-2-hydroxy-propoxy)phenyl]-1,3,5-triazin, 2-(2-Hydroxyphenyl)-4-(4-methoxyphenyl)-6-phenyl-1,3,5-triazin, 2-{2-Hydroxy-4-[3-(2-ethylhexyl-1-oxy)-2-hydroxypropyloxy]phenyl}-4,6-bis(2,4-dimethylphenyl)-1,3,5-triazin.

   Es können einzelne dieser Verbindungen oder Gemische derselben eingesetzt werden.
3. Geeignete Metalldesaktivatoren sind zum Beispiel N,N'-Diphenyloxamid, N-Salicylal-N'-salicyloylhydrazin, N,N'-Bis(salicyloyl)hydrazin, N,N'-Bis(3,5-di-tert-butyl-4-hydroxyphenylpropionyl)hydrazin, 3-Salicyloylamino-1,2,4-triazol, Bis(benzyliden)oxalyldihydräzid, Oxanilid, Isophthaloyldihydrazid, Sebacoylbisphenylhydrazid, N,N'-Diacetyladipoyldihydrazid, N,N'-Bis(salicyloyl)oxalyldihydrazid, N,N'-Bis(salicyloyl)thiopropionyldihydrazid. Es können einzelne dieser Verbindungen oder Gemische derselben eingesetzt werden.
4. Geeignete Peroxidfänger sind zum Beispiel Ester von β-Thiodipropionsäure, zum Beispiel der Lauryl-, Stearyl-, Myristyl- oder Tridecylester, Mercaptobenzimidazol oder das Zinksalz von 2-Mercaptobenzimidazol, Zinkdibutyldithiocarbamat, Dioctadecyldisulfid, Pentaerythrittetrakis-(dodecylmercapto)propionat. Es können einzelne dieser Verbindungen oder Gemische derselben eingesetzt werden.
5. Geeignete basische Costabilisatoren sind zum Beispiel Melamin, Polyvinylpyrrolidon, Dicyandiamid, Triallylcyanurat, Harnstoffderivate, Hydrazinderivate, Amine, Polyamide, Polyurethane, Alkalimetallsalze und Erdalkalimetallsalze höherer Fettsäuren, zum Beispiel Calciumstearat, Zinkstearat, Magnesiumbehenat, Magnesiumstearat, Natriumricinoleat und Kaliumpalmitat, Antimonpyrocatecholat oder Zinkpyrocatecholat. Es können einzelne dieser Verbindungen oder Gemische derselben eingesetzt werden.
6. Geeignete Keimbildner sind zum Beispiel anorganische Substanzen, wie Talk, Metalloxide, wie Titandioxid oder Magnesiumoxid, Phosphate, Carbonate oder Sulfate, vorzugsweise von Erdalkalimetallen; organische Verbindungen, wie Mono- oder Polycarbonsäuren und deren Salze, z.B. 4-tert-Butylbenzoesäure, Adipinsäure, Diphenylessigsäure, Natriumsuccinat oder Natriumbenzoat; polymere Verbindungen, wie ionische Copolymere (Ionomere). Besonders bevorzugt sind 1,3:2,4-Bis(3',4'-dimethylbenzyliden)sorbit, 1,3:2,4-Di(paramethyldibenzyliden)sorbit und 1,3:2,4-Di-(benzyliden)sorbit. Es können einzelne dieser Verbindungen oder Gemische derselben eingesetzt werden.
7. Geeignete Füllstoffe und Verstärkungsmittel sind zum Beispiel Calciumcarbonat, Silicate, Glasfasern, Glasballons, Asbest, Talk, Kaolin, Glimmer, Bariumsulfat, Metalloxide und -hydroxide, Ruß, Graphit, Wollastonit, Holzmehl und Mehle oder Fasern anderer Naturprodukte, synthetische Fasern. Es können einzelne dieser Verbindungen oder Gemische derselben eingesetzt werden.
8. Geeignete andere Additive sind zum Beispiel Weichmacher, Gleitmittel, Emulgatoren, Pigmente, Viskositätsmodifikatoren, Katalysatoren, Verlaufmittel, optische Aufheller, Flammschutzmittel, antistatische Mittel und Treibmittel.
9. Geeignete Benzofuranone und Indolinone sind zum Beispiel diejenigen, die in U.S. 4,325,863; U.S. 4,338,244.; U.S. 5,175,312; U.S. 5,216,052; U.S. 5,252,643; DE-A-4316611; DE-A-4316622; DE-A-4316876; EP-A-0589839 oder EP-A-0591102 offenbart sind, oder 3-[4-(2-Acetoxyethoxy)phenyl]-5,7-di-tert-butyl-benzofuran-2-on, 5,7-Di-tert-butyl-3-[4-(2-stearoyloxyethoxy)-phenyl]benzofuran-2-on, 3,3'-Bis[5,7-di-tert-butyl-3-(4-[2-hydroxyethoxy]phenyl)benzofuran-2-on], 5,7-Di-tert-butyl-3-(4-ethoxyphenyl)benzofuran-2-on, 3-(4-Acetoxy-3,5-dimethylphenyl)-5,7-di-tert-butylbenzofuran-2-on, 3-(3,5-Dimethyl-4-pivaloyloxyphenyl)-5,7-di-tert-butylbenzo-furan-2-on, 3-(3,4-Dimethylphenyl)-5,7-di-tert-butylbenzofuran-2-on, 3-(2,3-Dimethylphenyl)-5,7-di-tert-butylbenzofuran-2-on, Lacton-Antioxidantien wie 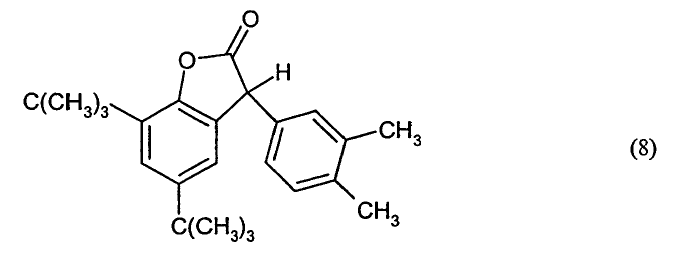
   Diese Verbindungen wirken beispielsweise als Antioxidantien. Es können einzelne dieser Verbindungen oder Gemische derselben eingesetzt werden.
10. Geeignete fluoreszierende Weichmacher sind die in "Plastics Additives Handbook", Hrsg. R. Gächter und H. Müller, Hanser Verlag, 3. Aufl., 1990, Seite 775-789 aufgeführten.
11. Geeignete flammhemmende Additive sind Phosphatester, d.h. Triphenylphosphat, Resorcindiphosphorsäureester, bromhaltige Verbindungen, wie bromierte Phosphorsäureester, bromierte Oligocarbonate und Polycarbonate, sowie Salze, wie C₄F₉SO₃⁻Na⁺.
12. Geeignete Schlagzähmacher sind Butadienkautschuk mit aufgepfropftem Styrol-Acrylnitril oder Methylmethacrylat, Ethylen-Propylen-Kautschuke mit aufgepfropftem Maleinsäureanhydrid, Ethyl- und Butylacrylatkautschuke mit aufgepfropftem Methylmethacrylat oder Styrol-Acrylnitril, interpenetrierende Siloxan- und Acrylat-Netzwerke mit aufgepfropftem Methylmethacrylat oder Styrol-Acrylnitril.
13. Geeignete Polymere sind SAN, ABS, PMMA, PTFE, PSU, PPS, Polyolefine, wie Polyethylen, Polypropylen und Ethylen-Propylen-Kautschuke, Epoxyharze, Polyester, wie PBT, PET, PCT, PCTG und PETG sowie andere im Grenzflächenverfahren erzeugte Polycarbonate.
14. Geeignete antistatische Mittel sind Sulfonatsalze beispielsweise Tetraethylammoniumsalze von C₁₂H₂₅SO³⁻ oder C₈F₁₇SO³⁻.
15. Geeignete Färbemittel sind Pigmente sowie organische und anorganische Farbstoffe.
16. Verbindungen, die Epoxygruppen enthalten, wie 3,4-Epoxycyclohexylmethyl-3,4-epoxycyclohexylcarboxylat, Copolymere von Glycidylmethacrylat und Epoxysilane.
17. Verbindungen, die Anhydridgruppen, wie Maleinsäureanhydrid, Bernsteinsäureanhydrid, Benzoesäureanhydrid und Phthalsäureanhydrid.
18. Als Stabilisatoren geeignete Phosphite und Phosphonites sind zum Beispiel Triphenylphosphit, Diphenylalkylphosphite, Phenyldialkylphosphite, Tris(nonylphenyl)phosphit, Trilaurylphosphit, Trioctadecylphosphit, Distearylpentaerythritdiphosphit, Tris(2,4-di-tert-butylphenyl)phosphit, Diisodecylpentaerythritdiphosphit, Bis(2,4-di-tert-butylphenyl)pentaerythritdiphosphit, Bis(2,6-di-tert-butyl-4-methylphenyl)pentaery-thritdiphosphit, Düsodecyloxypentaerythritdiphosphit, Bis(2,4-di-tert-butyl-6-methylphenyl)pentaerythritdiphosphit, Bis(2,4,6-tris(tert-butylphenyl)pentaerythritdiphosphit, Tristearylsorbittriphosphit, Tetrakis(2,4-di-tert-butylphenyl)-4,4'-biphenylendiphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis(2,4-di-tert-butyl-6-methylphenyl)methylphosphit, Bis(2,4-di-tert-butyl-6-methylphenyl)ethylphosphit, 6-Fluor-2,4,8,10-tetra-tert-butyl-12-methyldibenz[d,g]-1,3,2-dioxaphosphocin, 2,2',2"-Nitrilo[triethyltris(3,3', 5,5'tetra-tert-butyl-1,1'-biphenyl-2,2'-diyl)phosphit], 2-Ethyl-hexyl(3,3',5,5'-tetra-tert-butyl-1,1'-biphenyl-2,2'-diyl)phosphit, 5-Butyl-5-ethyl-2-(2,4,6-tri-tert-butylphenoxy)-1,3,2-dioxaphosphiran. Es können einzelne dieser Verbindungen oder Gemische derselben eingesetzt werden.

Besonders bevorzugt sind Tris(2,4-di-tert-butylphenyl)phosphit (Irgafos® 168, Ciba-Geigy), oder Triphenylphosphin.

Die Verbindungen der Gruppen 16 und 17 wirken als Schmelzestabilisatoren. Sie können einzeln oder in Gemischen eingesetzt werden.

Als Entformungsmittel werden vorzugsweise Ester ein- oder mehrwertiger Alkohole mit langkettigen Carbonsäuren verwandt wie Loxiol G32 oder Loxiol G33. Bevorzugt sind auch solche Entformungsmittel, die nicht vollständig verestert wurden, demnach freie OH-Gruppen aufweisen. Besonders bevorzugt sind (Teil-)Ester von gesättigten einwertigen Fettsäuren mit 16 bis 22 Kohlenstoffatomen mit Glycerin, Trimethylolpropan, Pentaerythrit oder ähnlichen mehrwertigen Alkoholen. Insbesondere Glycerinmonostearat (GMS) und Glycerinmonopalmiat. Weiterhin bevorzugt sind Pentaerythrittetrastearat (PETS),

Solche gesättigten einwertigen Fettsäureester des Glycerins werden alleine oder als Mischungen mit zwei oder mehr Komponenten eingesetzt. Die gesättigten Monoester des Glycerins werden üblicherweise über die Umesterung von hydriertem tierischem oder pflanzlichem Öl mit Glycerin hergestellt. Obwohl das Reaktionsprodukt auch andere Ester als die Glycerinester enthalten kann, wird es als Entformungsmittel eingesetzt. Beispielsweise kann die Mischung kleine oder größere Anteile von Diglyceriden und Triglyceriden enthalten.

Das Optimum der Entformungsmittelmenge bei der Herstellung von CD's und anderen optischen Speichermedien (DVD's etc.) ist einerseits durch eine ausreichende entformende Wirkung, andererseits durch Belagsbildung auf dem Werkzeug bestimmt. Üblicherweise eingesetzte Konzentrationen liegen zwischen 50 bis 1000 ppm, vorteilhafter zwischen 100 und 500 ppm an Entformungsmittel. Für die übrigen Anwendungen von Polycarbonat liegen die Konzentrationen bei 100 - 10 000 ppm, bevorzugt bei 2 000 -7 000 ppm.

Als Thermostabilisatoren werden beispielsweise, aber nicht beschränkend, spezielle Phosphite verwendet, welche sowohl aromatische wie aliphatische Reste in einem Molekül besitzen. Es sind Verbindungen der folgenden Struktur: 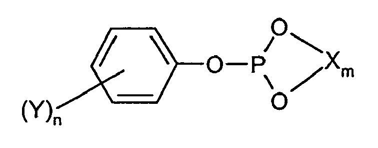 worin
- n: die Zahl 0-5, bevorzugt 1-3 darstellt und ganz besonders bevorzugt 3 darstellt,
- Y: jeweils unabhängig voneinander Alkyl oder gegebenenfalls substituiertes Aryl, bevorzugt C₁-C₄-Alkyl, besonders bevorzugt Methyl, sec.-Butyl und tert.-Butyl bedeutet,
- m: die Zahl 1-3, bevorzugt 3 darstellt und
- X: jeweils voneinander unabhängig für einen gegebenenfalls substituierten Methylenrest steht, wobei mindestens ein Methylenrest vollständig substituiert sein muss und die Substituenten unabhängig voneinander aus der Gruppe C₁-C₂₀-Alkyl ausgewählt sind oder aber die zwei Substituenten an einem vollständig substituierten Methylenrest gemeinsam für einen Rest 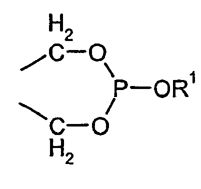
stehen,
in welchem R¹ aus der Gruppe C₁-C₁₈-Alkyl, C₃-C₁₂-Cycloalkyl, C₆-C₃₀-Alkaryl und Aryl, wobei diese Reste wiederum durch 1 - 4 O-Alkylen-O und/oder Carbonsäureester-COO-Reste substituiert sein können; C₂-C₁₈-Polyhydroxyalkyl mit 2 bis 10 Hydroxylgruppen; C₂-C₁₈-Polyphenylreste mit 2 bis zehn phenolischen OH-Gruppen, ausgewählt sind.

Bevorzugt sind dabei Verbindungen der Formel 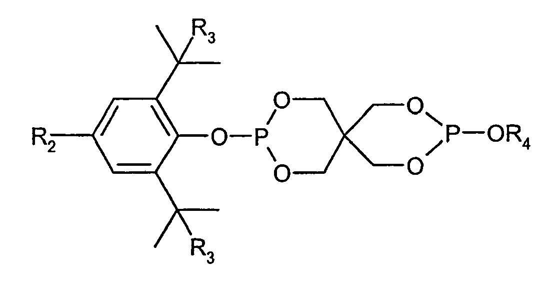 in welcher
- R₂: für C₁-C₆-Alkyl;
- R₃: für Methyl oder Ethyl und
- R₄: aus der Gruppe C₁-C₁₈-Alkyl, C₃-C₁₂-Cycloalkyl, C₆-C₃₀-Alkaryl und Aryl, wobei diese Reste wiederum durch 1 - 4 O-Alkylen-O und/oder Carbonsäureester-COO-Reste substituiert sein können; C₂-C₁₈ -Polyhydroxyalkyl mit 2 bis 10 Hydroxylgruppen; C₂-C₁₈-Polyphenylreste mit 2 bis 10 phenolischen OH-Gruppen, ausgewählt ist.

Ebenfalls bevorzugt sind Verbindungen der Formel 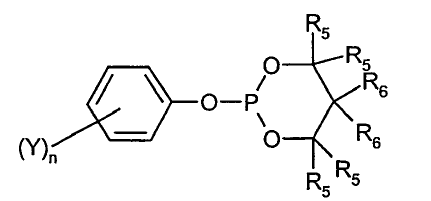 worin Y und n die oben genannten Bedeutungen haben und
- R₅: unabhängig voneinander aus der Gruppe Wasserstoff und C₃-C₂₀-Alkyl ausgewählt wird, bevorzugt steht dabei mind. ein R₅ für Alkyl,
- R₆: unabhängig voneinander für C₁-C₁₀-Alkyl stehen.

Besonders bevorzugt sind Verbindungen der Formel 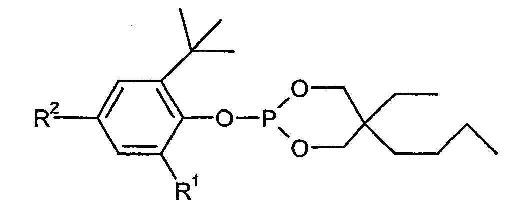 wobei R¹ und R² für Methyl, sec-Butyl oder tert-Butyl stehen.

Ebenfalls besonders bevorzugt sind ausserdem die in EP A1 0 038 876 auf S. 16-20 definierten Verbindungen sowie das in der gleichen Schrift auf Seite 21 genannte Beispiel.

Ganz besonders bevorzugt ist (2,4,6-Tri-t-butylphenyl)-(2-butyl-2-ethyl-propan-1,3-diyl)-phosphit, welches folgende Struktur aufweist:

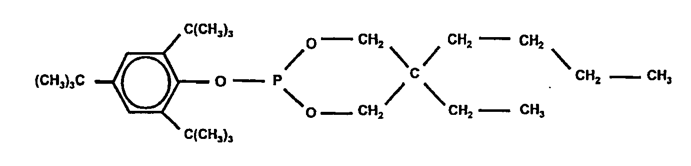

Die Phosphite können allein, jedoch auch in Kombination mit anderen Phosphorverbindungen eingesetzt werden, wobei die anderen Phosphorverbindungen auch solche sein können, die eine andere Oxidationszahl des Phosphors besitzen. Demnach können z.B. Kombinationen der erfindungsgemäßen Phosphite mit anderen Phosphiten, mit Phosphinen, z.B. Triphenylphosphin, mit Phosphoniten, mit Phosphaten, mit Phosphonaten usw. eingesetzt werden.

Die eingesetzten Phosphite sind generell bekannt oder analog bekannter Phosphite herstellbar, (2,4,6-Tri-t-butylphenyl)-(2-butyl-2-ethyl propan-1,3-diyl)-phosphit ist z.B. beschrieben in der EP-A 702018 und EP 635514.

Die erfindungsgemäßen Polymermischungen enthalten die Phosphorverbindung im Allgemeinen zu einem Anteil von 10 - 5 000 ppm, bevorzugt 10 - 1 000 ppm, besonders bevorzugt 20 - 500ppm, ganz besonders bevorzugt zwischen 50 und 250 ppm.

Der Zusatz der Entformungsmittel, der Phosphorverbindung und der erfindungsgemäßen Formale zu den thermoplastischen Formmassen erfolgt beispielhaft und vorzugsweise, indem man sie nach der Herstellung und während der Aufarbeitung der Polycarbonate, z.B. durch Zugabe zu der Polycarbonat-Polymerlösung, oder einer Schmelze der thermoplastischen Formmassen zudosiert. Weiterhin ist es auch möglich, die Komponenten unabhängig voneinander in verschiedenen Arbeitsschritten zuzudosieren, z.B. eine der Komponenten während der Aufarbeitung der Polymerlösung und die andere(n) Komponte(n) in der Schmelze, solange gewährleistet ist, dass alle Komponenten bei der Herstellung der Endprodukte (Formkörper) enthalten sind.

Der Fachmann wird für Anwendungen im Bereich der CD, DVD und anderer optischer Aufzeichnungsmedien aus den oben genannten Additiven selbstverständlich geeignete, die Transparenz nicht beeinträchtigende Additive auswählen.

Ganz besonders geeignete Additive sind IRGANOX 1076 ®, s.o. und Benzotriazole der Gruppe 2.1 (sog. Tinuvine), insbesondere in Mischung miteinander sowie Triphenylphosphin (TPP).

Die erfindungsgemäßen Formmassen werden in der für Polycarbonate bekannten Weise zur Herstellung von Formkörpern, vorzugsweise optischen Medien, verwandt. Insbesondere zur Herstellung von Compact Discs und DVD's sowie einmal- oder mehrfach beschreibbaren bzw. löschbaren optischen Medien. Die beschreibbaren Schichten bestehen dabei insbesondere aus Farbstoffen oder metallischen Schichten, wobei letztere als Aufzeichnungsprinzip den Wechsel vom amorphen in den kristallinen Zustand benutzen oder magnetische Eigenschaften besitzen.

Diese Herstellung der optischen Medien erfolgt vorzugsweise aus den fertig hergestellten, erfindungsgemäßen Formmassen, die beispielsweise als Granulat anfallen. Die Herstellung der optischen Medien kann aber auch durch Einarbeitung der Komponenten zu reinen oder handelsüblichen Polycarbonaten und/oder zu den bei der Herstellung von Formkörpern aus Polycarbonaten üblichen Zusätzen erfolgen.

Ein weiterer Gegenstand der Erfindung sind demnach Formkörper, wie insbesondere optische Datenträger, bevorzugt Compact Discs und DVD's die aus den erfindungsgemäßen thermoplastischen Formmassen erhältlich sind.

Die erfindungsgemäßen thermoplastischen Formmassen haben den Vorteil, dass sie eine geringere Wasseraufnahme und damit eine verbesserte Dimensionsstabilität aufweisen. Außerdem zeichnen sie sich durch ein verbessertes Fließverhalten aus, da sie eine geringere Schmelzviskosität aufweisen.

Die nachfolgende Beispiele dienen zur Erläuterung der Erfindung. Die Erfindung ist nicht auf die Beispiele beschränkt.

### Beispiele:

### Synthese der aromatischen Formale

### Beispiel 1:

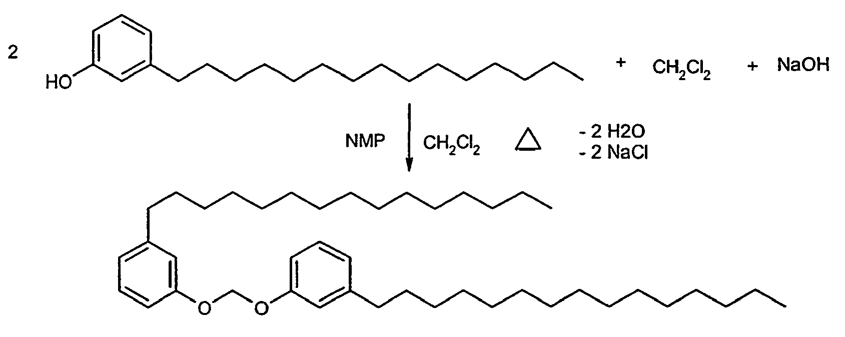

50,0 g (0,16 mol) destilliertes 3-Pentadecylphenol und 16,0 g (0,40 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 44,8 g (90,2 % der Theorie) eines wachsartigen Feststoffes.
Analytik:
¹H-NMR (400 MHz, TMS, CDCl₃) δ = 7,2-6,82 (m, 8H), 5,68 (s, 2H), 2,58-2,54 (m, 4H), 1,62-1,57 (m, 4H), 1,39-1,20 (m, 48H), 0,89-0,86 (t, 6H).

### Beispiel 2:

Analog Beispiel 1, jedoch 4-fache Ansatzgröße.
Ausbeute: 181,6 g (91,4 % der Theorie)
Analytik: analoges NMR-Spektrum, wie unter Beispiel 1

### Beispiel 3:

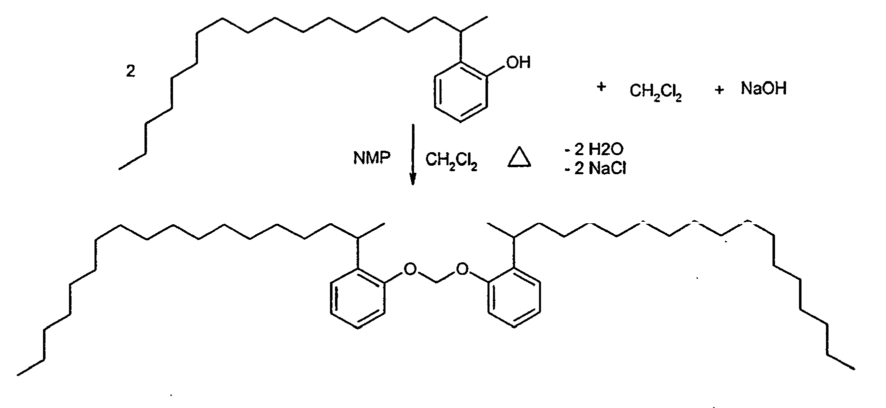

50,0 g (0,14 mol) Octadecylphenol und 14,4 g (0,36 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 42,0 g (85,1 % der Theorie) eines wachsartigen Feststoffes.
Analytik:
¹H-NMR (400 MHz, TMS, CDCl₃) δ = 7,22-6,97 (m, 8H), 5,7 (s, 2H), 3,15-3,05 (m, 2H), 1,55-0,9 (m, 36H), 0,85-0,81 (t, 6H).

### Beispiel 4:

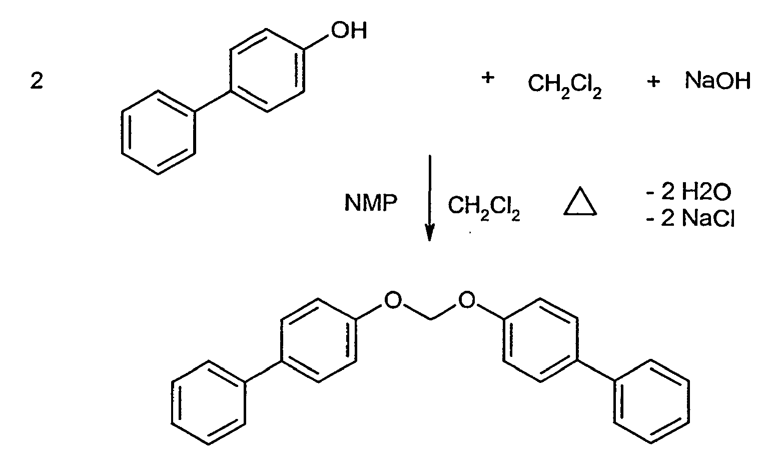

23,8 g (0,14 mol) 4-Hydroxybiphenyl und 14,4 g (0,36 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 21,7 g (87,9 % der Theorie) eines weißen Feststoffes.
Analytik:
¹H-NMR (400 MHz, TMS, CDCl₃) δ = 7,65-7,5 (m, 8H), 7,45-7,35 (m, 4H), 7,35-7,25 (m, 2H), 7,25-7,15 (m, 4H), 5,8 (s, 2H).

### Beispiel 5:

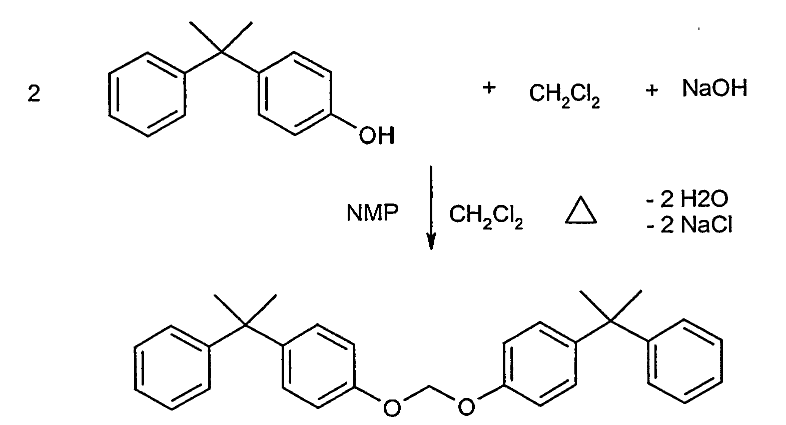

29,7 g (0,14 mol) 4-Cumylphenol und 14,4 g (0,36 mol) Natriumhydroxid (Microprills der Fa, Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 21,5 g (70,3 % der Theorie) eines weißen Feststoffes.
Analytik:
¹H-NMR (400 MHz, TMS, CDCl₃) δ = 7,28-7,19 (m, 8H), 7,19-7,09 (m,6H), 7,05-6,95 (m, 4H), 5,68 (s, 2H), 1,65 (s, 12 H).

### Beispiel 6:

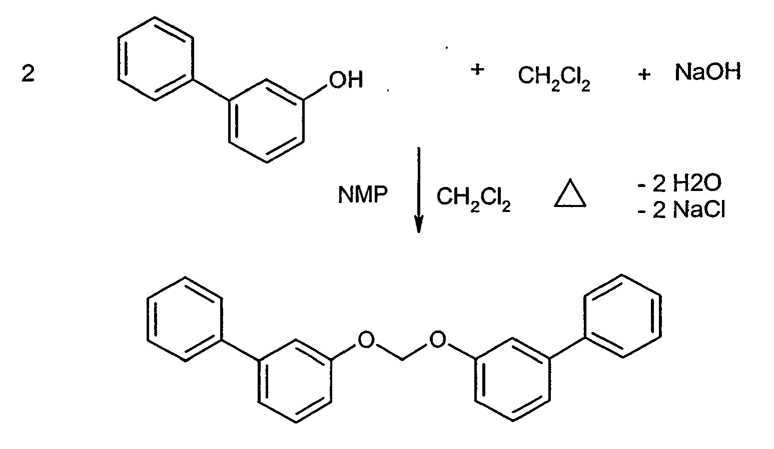

23,8 g (0,14 mol) 3-Hydroxybiphenyl und 14,4 g (0,36 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 14,2 g (57,6 % der Theorie) eines weißen Feststoffes.
Analytik:
¹H-NMR (400 MHz, TMS, CDCl₃) δ = 7,6-7,5 (m, 4H), 7,5-7,2 (m, 12H), 7,15-7,05 (m, 2H), 5,85 (s, 2H).

### Beispiel 7:

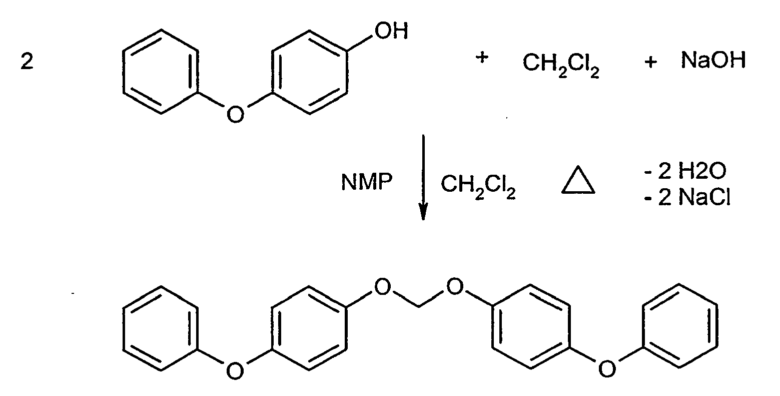

24,2 g (0,13 mol) 4-Hydroxydiphenylether und 13,0 g (0,325 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in einem Methanol/Wassergemisch (1:1) ausgefällt. Das erhaltene Rohprodukt wurde abschließend mehrmals mit Wasser nachgewaschen und schließlich bei 80°C im Vakuumtrockenschrank getrocknet. Man erhielt 21,0 g (84,0 % der Theorie) eines weißen Feststoffes.
Analytik:
¹H-NMR (400 MHz, TMS, CDCl₃) δ = 7,35-7,22 (m, 4H), 7,15-7,02 (m, 6H), 7,02-6,9 (m, 8H), 5,65 (s, 2H).

### Beispiel 8:

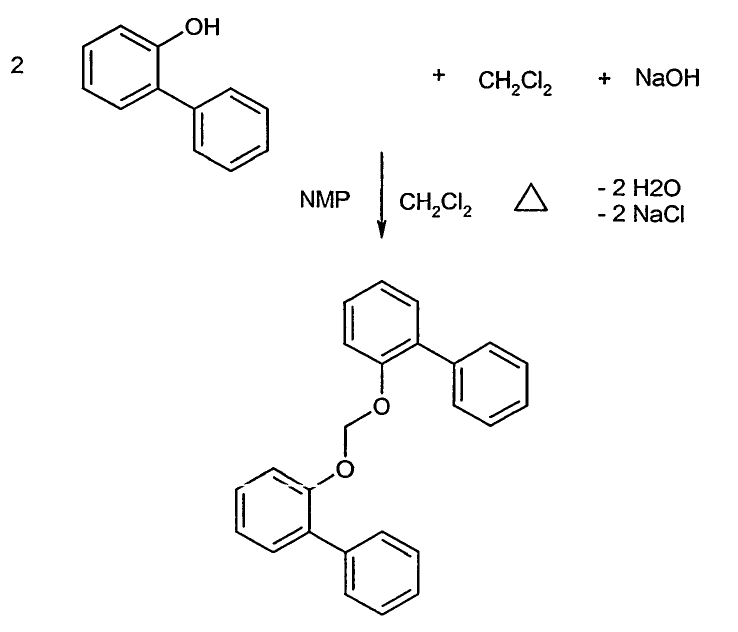

23,8 g (0,14 mol) 4-Cumylphenol und 14,4 g (0,36 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 13,0 g (52,7 % der Theorie) eines weißen Feststoffes.
Analytik:
¹H-NMR (400 MHz, TMS, CDCl₃) δ = 7,45-7,15 (m, 16H), 7,15-7,05 (m, 2H), 5,58 (s, 2H).

### Beispiel 9:

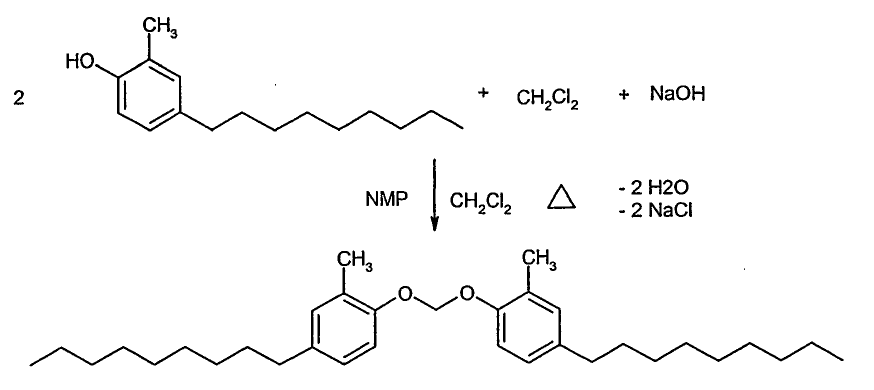

50,0 g (0,21 mol) 2-Methyl-4-nonylphenol und 21,3 g (0,53 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 9,4 g (18,6 % der Theorie) einer hochviskosen Substanz.
Analytik:
¹H-NMR (400 MHz, TMS, CDCl₃) δ = 7,1-6,9 (m, 6H), 5,65 (s, 2H), 2,12 (s, 6H), 1,7-0,45 (m, 38H).

### Beispiel 10:

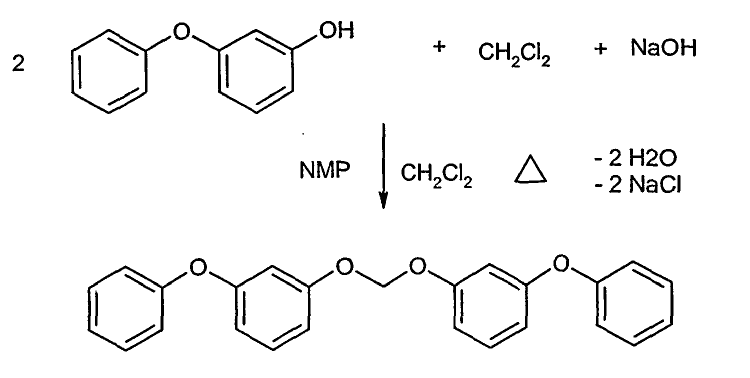

17,4 g (0,093 mol) 3-Hydroxydiphenylether und 9,3 g (0,23 mol) Natriumhydroxid (Microprills der Fa. Riedel) werden zu einer Mischung von 100 ml Methylenchlorid und 180 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Anschließend wurde die organische Phase eingeengt. Das erhaltene Rohprodukt wurde abschließend mehrmals mit Wasser nachgewaschen und schließlich unter Hochvakkuum bei 160°C getrocknet. Man erhielt 15,7 g (90,7 % der Theorie) einer hochviskosen Substanz.
Analytik:
¹H-NMR (400 MHz, TMS, CDCl₃) δ = 7,45-7,35 (m,4H), 7,35-7,2 (m, 2H), 7,2-7,1 (m,2H), 7,1-7,0 (m, 4H), 6,9-6,8 (m, 2H), 6,8-6,7 (m, 2H), 6,7-6,6 (m, 2H), 5,82 (s, 2H).

### Beispiel 11:

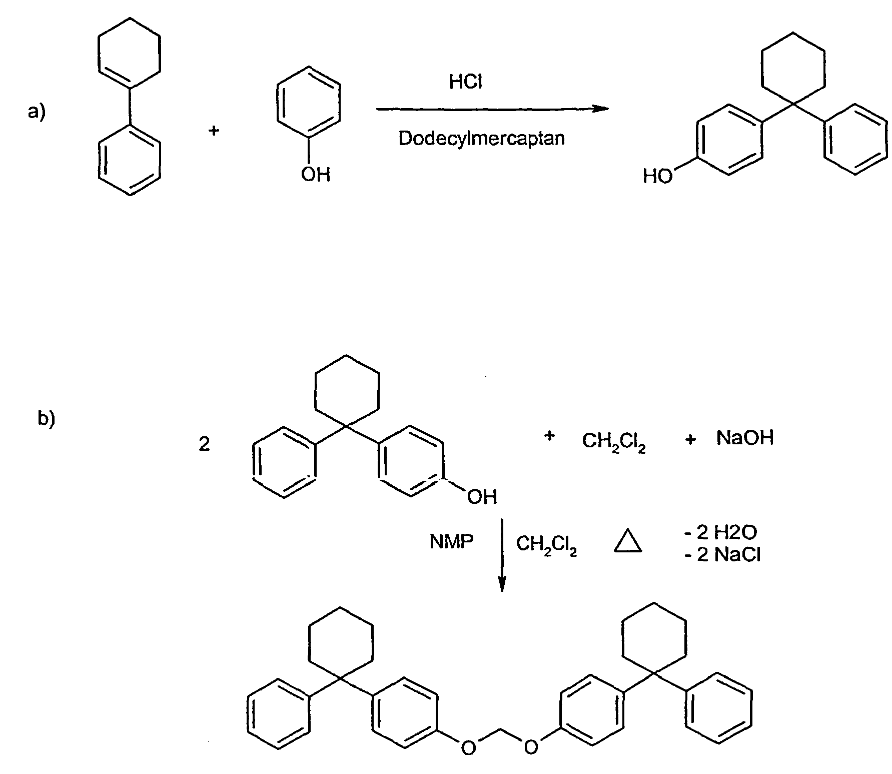

a) Man legt 24 g (0,15 mol) 1-Phenyl-1-cyclohexen (Fa. Aldrich) und in großem Überschuss 56,5 g (0,60 mol) frisch destilliertes Phenol vor und versetzt den Reaktionsansatz mit 0,3 g (0,0015 mol) Dodecylmercaptan als Katalysator. Bei einer Temperatur von 30°C beginnt man mit dem Einleiten von gasförmigen Chlorwasserstoff. Dies hält man insgesamt für 20 Minuten aufrecht. Nach Verfestigung des Ansatzes wurde im Wasserstrahlvakuum das überschüssige Phenol abdestilliert. Die letzten Reste an Phenol ließen sich bei 120°C im Hochvakuum abdestillieren. Man erhält 36,5 g (96,4 % der Theorie) eines weißen Feststoffes.
   Analytik:
   ¹H-NMR (400 MHz, TMS, CDCl₃) δ = 9,12 (s, 1H), 7,3-7,15 (m, 4H), 7,15-7,0 (m, 3H), 6,7-6,6 (m, 2H), 2,2 (m, 4H), 1,45 (m, 6H).
b) 5 g (0,0198 mol) Produkt aus Beispiel 11a) und 1,98 g (0,05 mol) Natriumhydroxid (Microprills der Fa. Riedel) werden zu einer Mischung von 30 ml Methylenchlorid und 55 ml N-Methylpyrrolidon (NMP) hinzugegeben. Anschließend wurde 1 Stunde am Rückfluss gekocht.

Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 3,7 g (72,3 % der Theorie) eines weißen Feststoffes.
Analytik:
¹H-NMR (400 MHz, TMS, CDCl₃) δ = 7,3-7,2 (m, 8), 7,2-7,15 (m, 4H), 7,15-7,05 (m, 2H9, 7,0-6,9 (m, 4H), 5,65 (s, 2H), 2,25 (m, 8H), 1,65-1,45 (m, 12H).

### Beispiel 12:

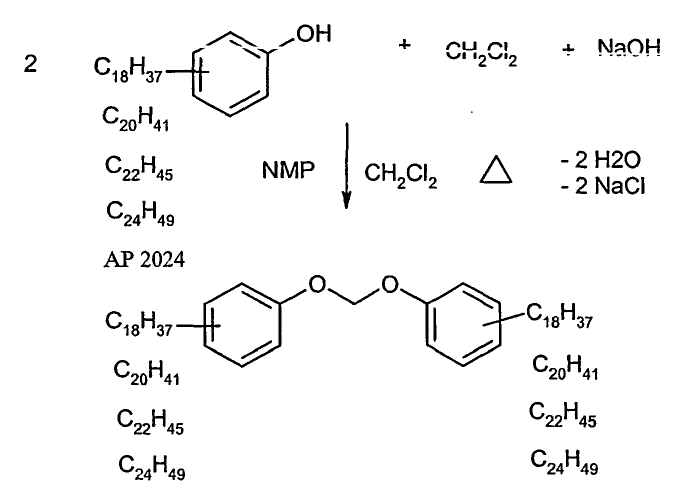

50,0 g AP 2024 (Gemisch von Phenolen mit den o.g. Resten R¹, Fa. Idemitsu Petrochemicals Co. Ltd., Tokyo, Japan) und 13,2 g (0,33 mol) Natriumhydroxid (Microprills der Fa. Riedel) werden zu einer Mischung von 125 ml Methylenchlorid und 225 ml N-Methylpyrrolidon (NMP) hinzugegeben. Anschließend wurde 1 Stunde am Rückfluss gekocht. Nach Abkühlen des Reaktionsansatzes wurden Methylenchlorid und Wasser hinzugegeben, bis eine ausreichende Phasentrennung erreicht war. Die organische Phase wurde abgetrennt und mehrmals mit Wasser bis zum Neutralpunkt gewaschen. Die getrocknete organische Phase wurde anschließend in Methanol ausgefällt. Das erhaltene Rohprodukt wurde abschließend mehrmals mit Methanol nachgewaschen und schließlich bei 80°C im Vakuumtrockenschrank getrocknet. Man erhielt 30,5 g einer hochviskosen Substanz.
Analytik:
¹H-NMR (400 MHz, TMS, CDCl₃) δ = 7,2-6,9 (m, Hₐᵣ), 5,75-5,65 (m, H _{OCH2}), 1,7-0,7 (m, Hₐₗₖ).

Die so erhaltenen Formale werden als Additiv in folgenden Polycarbonattypen getestet:

Makrolon^{®} CD2005 (Bayer AG, globaler Polycarbonattyp für optische Speichermedien auf Basis von Bisphenol A, niedrigviskos, MFR 63 g/10 min, leicht entformbar, Spritzgiessen)

Apec 1800 (Bayer AG, Co-Polycarbonattyp auf Basis von Bisphenol A und TMC-Bisphenol; Basistyp Erweichungstemperatur (VST/B 120)=185°C)

Die Bestimmung des Wassergehaltes der Polycarbonate erfolgt nach Lagerung im Feuchtklima bei 95 % relativer Luftfeuchte und 30°C Lagertemperatur. Der Wassergehalt wird unmittelbar vor sowie nach 7 und 14 Tagen Lagerung im Feuchtklima mittels quantitativer Karl-Fischer-Titration (Coulometrische Titration) bestimmt.

| **Wasseraufnahme [Gew.-%] von Polycarbonat mit Additiven** | | | | | | | | | | | | |
|---|---|---|---|---|---|---|---|---|---|---|---|---|
| | | Struktur | | | Polymer | | Beispiel 1 [Gew.- %] | | Tg [°C] | direkt | nach 7 Tagen | nach 14 Tagen |
| 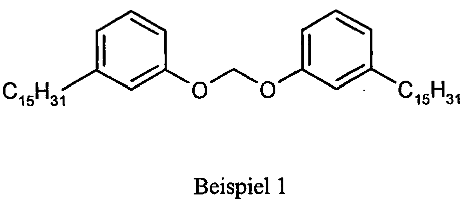 | | | | | Makrolon CD2005 | mit | 1.0 | von | 135 | 0.07% | 0.20..0.21% | 0.15..0.19% |
| | | | | | Makrolon CD2005 | mit | 2.0 | von | 128 | 0.03..0.04% | 0.18..0.19% | 0.14..0.23% |
| | | | | | Makrolon CD2005 | mit | 3.0 | von | 123 | 0.03/0.05 | 0.17/0.23 | 0.17/0.24 |
| | | | | | Apec 1800 | mit | 1.0 | von | 176 | 0.04 | 0.26/0.30 | 0.26/0.29 |
| | | | | | Apec 1800 | mit | 2.0 | von | 165 | 0.03/0.04 | 0.20/0.26 | 0.19 / 0.28 |
| | | | | | Apec 1800 | mit | 3.0 | von | 157 | 0.02/0.03 | 0.23 / 0.25 | 0.23/0.26 |
| 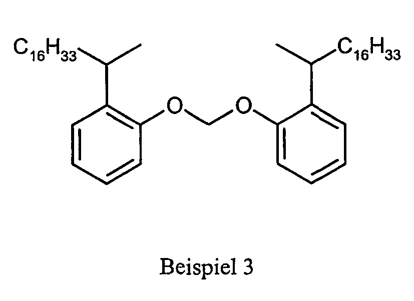 | | | | | Makrolon CD2005 | mit | 1.0 | von | 136 | 0.06% | 0.18..0.20% | 0.19..0.27% |
| | | | | | Makrolon CD2005 | mit | 2.0 | von | 130 | 0.07% | 0.14..0.26% | 0.15..0.24% |
| | | | | | Makrolon CD2005 | mit | 3.0 | von | 126 | 0.06% | 0.15..0.21% | 0.15..0.16% |
| | | | | | Apec 1800 | mit | 1.0 | von | 175 | 0.06..0.07% | 0.26..0.29% | 0.23..0.26% |
| | | | | | Apec 1800 | mit | 2.0 | von | 166 | 0.04..0.05% | 0.27..0.29% | 0.21..0.24% |
| | | | | | Apec 1800 | mit | 3.0 | von | 158 | 0.05% | 0.21..0.25% | 0.21..0.23% |
| 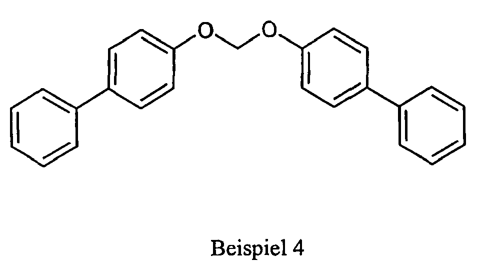 | | | | | Makrolon CD2005 | mit | 1.0 | von | 137 | 0.04 | 0.21/0.24 | 0.20/0.28 |
| | | | | | Makrolon CD2005 | mit | 2.0 | von | 132 | 0.05 | 0.20/0.22 | 0.21/0.29 |
| | | | | | Makrolon CD2005 | mit | 3.0 | von | 128 | 0.02 / 0.03 | 0.17 / 0.25 | 0.17 / 0.28 |
| | | | | | Apec 1800 | mit | 1.0 | von | 176 | 0.04 | 0.31 / 0.32 | 0.28/0.33 |
| | | | | | Apec 1800 | mit | 2.0 | von | | | | |
| | | | | | Apec 1800 | mit | 3.0 | von | | | | |
| 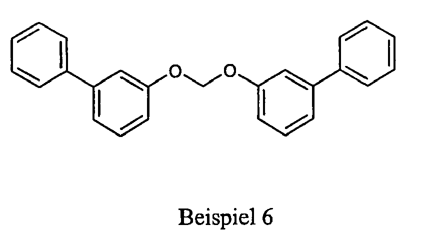 | | | | | | | | | | | | |
| | | | | | Makrolon CD2005 | mit | 3.0 | von | 127 | 0.03 | 0.12/0.25 | 0.15/0.23 |
| | | | | | | | | | | | | |
| | | | | | | | | | | | | |
| | | | | | Apec 1800 | mit | 3.0 | von | 165 | 0.03 | 0.23/0.26 | 0.23/0.25 |
| 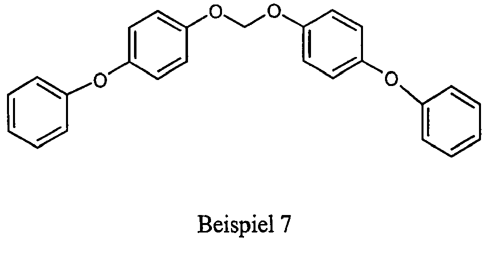 | | | | | | | | | | | | |
| | | | | | | | | | | | | |
| | | | | | Makrolon CD2005 | mit | 3.0 | von | 130 | 0.03 | 0.18/0.23 | 0.14/0.20 |
| | | | | | | | | | | | | |
| | | | | | | | | | | | | |
| | | | | | Apec 1800 | mit | 3.0 | von | 162 | 0.003 | 0.19/0.25 | 0.17/0.27 |

Auf analoge Weise wurden die folgenden Formale eingesetzt.

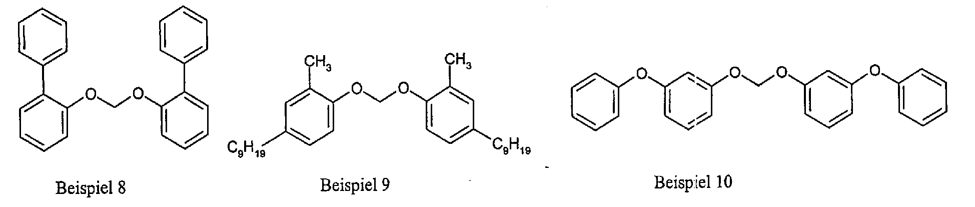

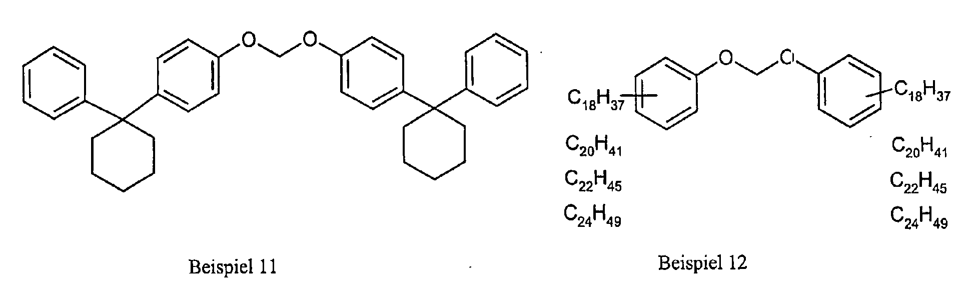

Die Versuchsergebnisse zeigen die überraschende und vollkommen unerwartete Wirkung der erfindungsgemäßen Verbindungen als die Wasseraufnahme senkende Additive in Polycarbonat. Die nicht entsprechend ausgerüsteten Polycarbonate zeigen im Vergleich dazu Wasseraufnahmen von 0,32-0,34 % für CD 2005 sowie 0,4 % für Apec 1800.

## Patentansprüche

1. Zusammensetzung enthaltend mindestens ein Polycarbonat und mindestens ein aromatisches Formal der folgenden Struktur 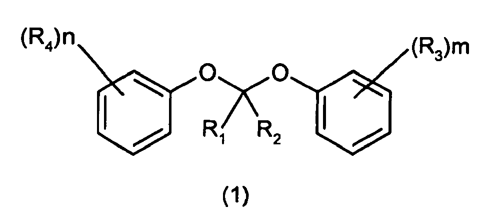 worin R₁ und R₂ für Wasserstoff oder Phenyl, R₃ und R₄ für Wasserstoff, lineare oder verzweigte C₁-C₄₀-Alky oder Alkyloxy I, gegebenenfalls substituiertes Aryl oder Aryloxy oder Aralkyl,n und m unabhängig voneinander für eine ganze Zahl zwischen 0 und 5 stehen, wobei es sich auch um Isomerengemische handeln kann.

2. Zusammensetzung nach Anspruch 1, enthaltend zusätzlich als Entformungsmittel Ester mehrwertiger Alkohole mit langkettigen Carbonsäuren sind, wobei die Ester mindestens noch eine freie OH-Gruppe enthalten.

3. Zusammensetzung gemäß den vorangehenden Ansprüchen, wobei das Polycarbonat ein Molekulargewicht von 15 000 bis 35 000 aufweist.

4. Zusammensetzung gemäß den vorangehenden Ansprüchen, enthaltend die aromatischen Formale zu einem Anteil von 10 - 60 000 ppm.

5. Zusammensetzung gemäß den vorangehenden Ansprüchen, enthaltend 0,01 bis 1,5 Gew-% an Entformungsmittel.

6. Zusammensetzung gemäß den vorangehenden Ansprüchen, enthaltend einen Thermostabilisator.

7. Verwendung der Zusammensetzungen gemäß der vorangehenden Ansprüche zur Herstellung von Formkörpern.

8. Optische Datenträger hergestellt aus Zusammensetzungen der Ansprüche 1 bis 7.

9. Verbindungen der Formel n 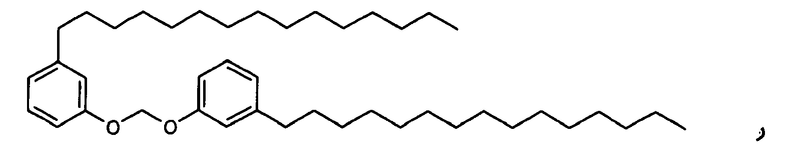 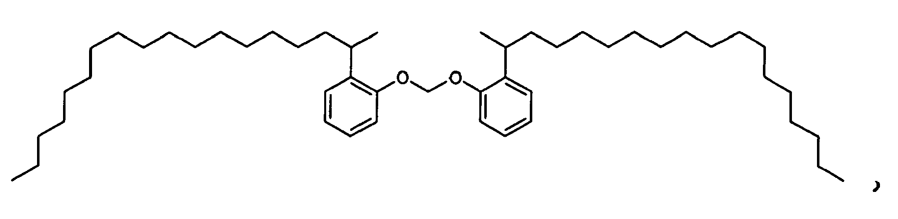 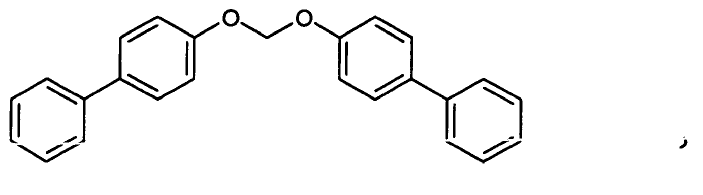 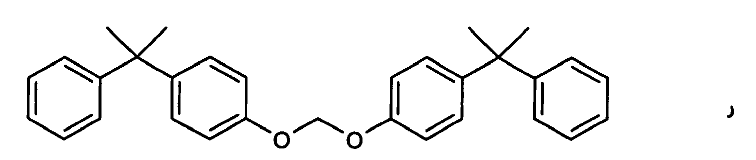 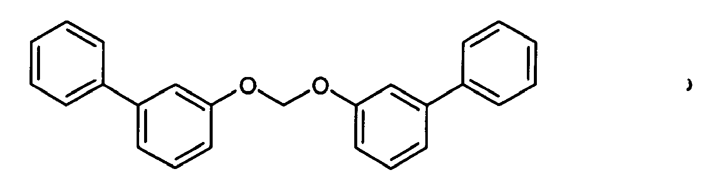 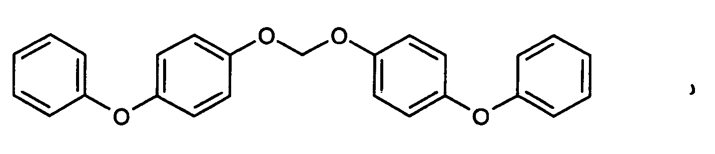 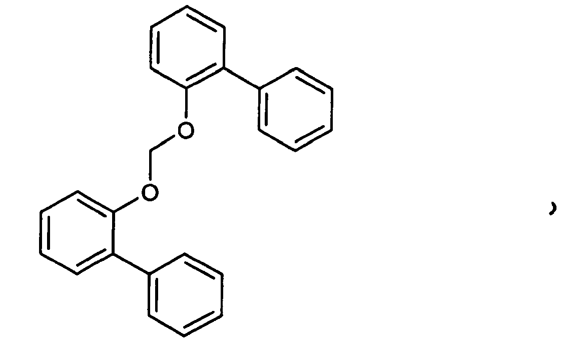 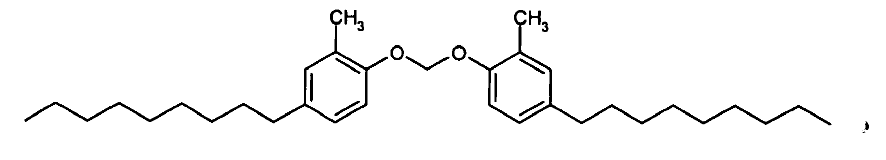 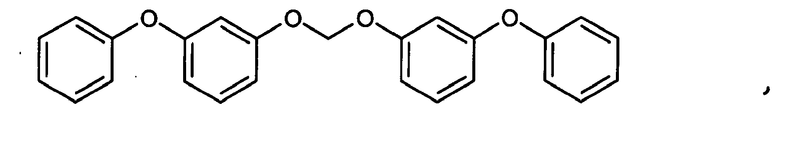 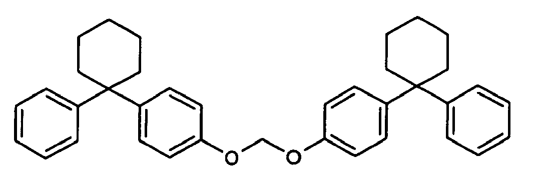 und

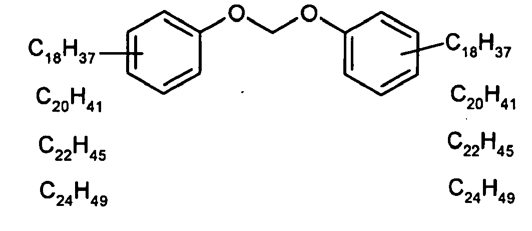

## Claims

1. Composition comprising at least one polycarbonate and at least one aromatic formal of the following structure 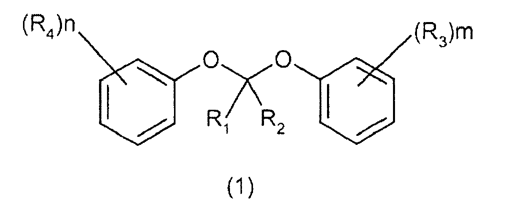 wherein R1 and R2 represent hydrogen or phenyl, R3 and R4 represent hydrogen, linear or branched C1-C40-alkyl or -alkoxy, optionally substituted aryl or aryloxy, or aralkyl and n and m independently of one another represent an integer between 0 and 5, it also being possible for these to be isomer mixtures.

2. Composition according to claim 1, wherein this additionally comprises, as mould release agents, esters of polyhydric alcohols with long-chain carboxylic acids, the esters still containing at least one free OH group.

3. Composition according to the preceding claims, wherein the polycarbonate has a molecular weight of 15,000 to 35,000.

4. Composition according to the preceding claims, comprising the aromatic formals in general in a content of 10 - 60,000 ppm.

5. Composition according to the preceding claims, comprising 0.01 to 1.5 wt.% of mould release agent.

6. Composition according to the preceding claims, comprising a heat stabilizer.

7. Use of the compositions according to the preceding claims for the production of shaped articles.

8. Optical data carriers produced from compositions of claims 1 to 7.

9. Compounds of the formulas 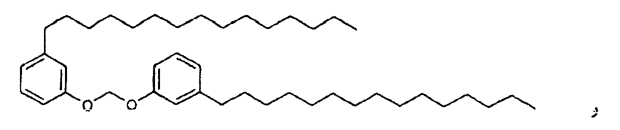 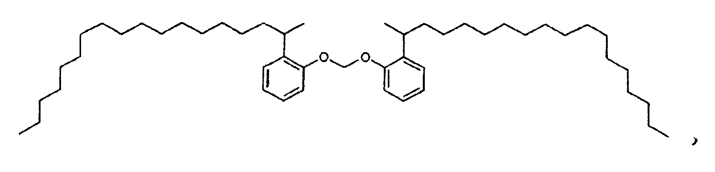 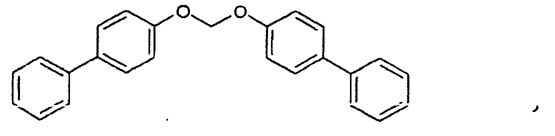 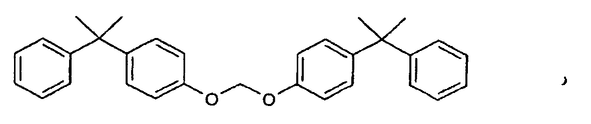 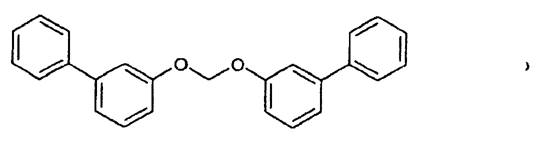 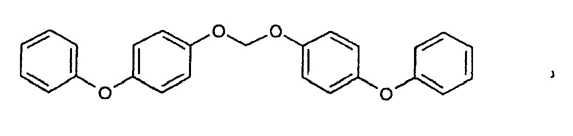 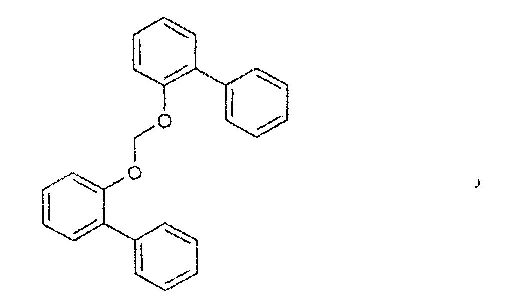 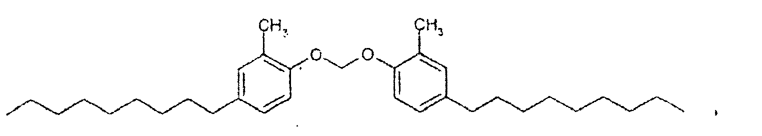 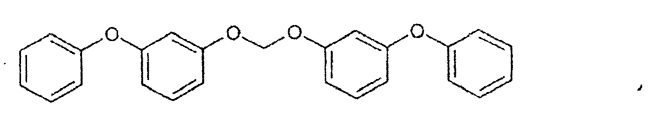 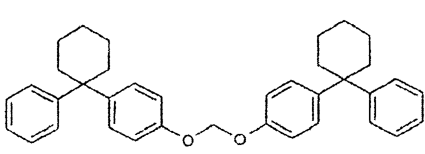 and

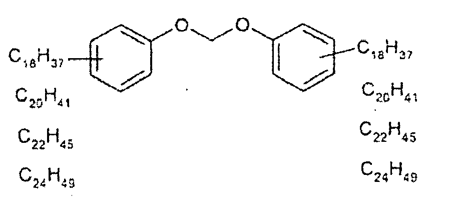

## Revendications

1. Composition contenant au moins un polycarbonate et au moins un formal aromatique à la structure suivante 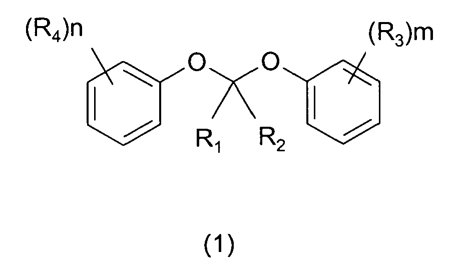 dans laquelle R₁ et R₂ représentent l'hydrogène ou un groupe phényle, R₃ et R₄ représentent l'hydrogène, des groupes alkyle ou alkyloxy linéaires ou ramifiés en C₁-C₄₀, des groupes aryle ou aryloxy ou aralkyle éventuellement substitués, les indices n et m sont égaux chacun, indépendamment les uns des autres, à un nombre entier allant de 0 à 5, et il peut s'agir d'un mélange d'isomères.

2. Composition selon la revendication 1 contenant en outre en tant qu'agents de démoulage, des esters d'alcools polyvalents et d'acides carboxyliques à longue chaîne, ces esters contenant encore au moins un groupe OH libre.

3. Composition selon les revendications qui précèdent, dans laquelle le polycarbonate a un poids moléculaire de 15 000 à 35 000.

4. Composition selon les revendications qui précèdent, contenant les formals aromatiques en proportion de 10 à 60 000 ppm.

5. Composition selon les revendications qui précèdent, contenant 0,01 à 1,5 % d'agents de démoulage.

6. Composition selon les revendications qui précèdent, contenant un stabilisant contre la chaleur.

7. Utilisation des compositions selon les revendications qui précèdent pour la fabrication d'objets moulés.

8. Banques de données optiques fabriquées à partir des compositions des revendications 1 à 7.

9. Composés de formules 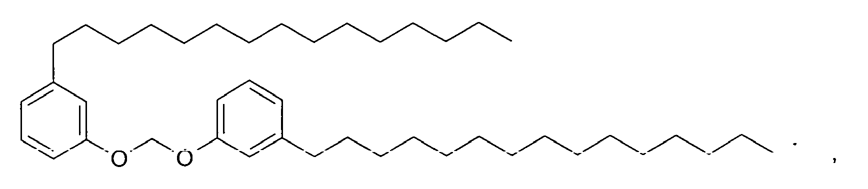 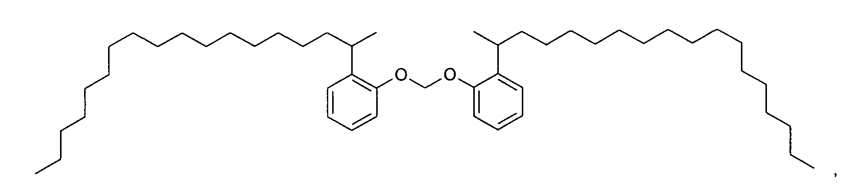 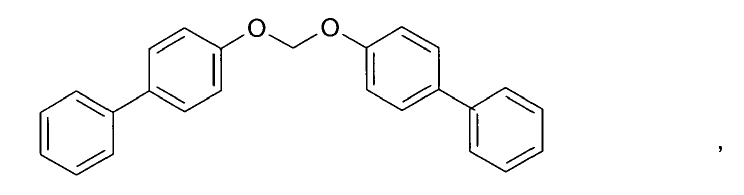 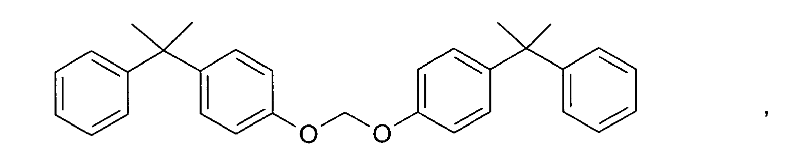 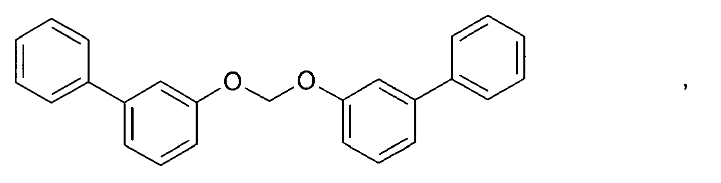 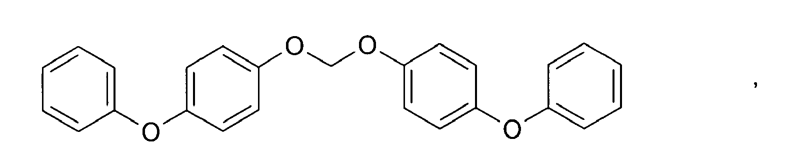 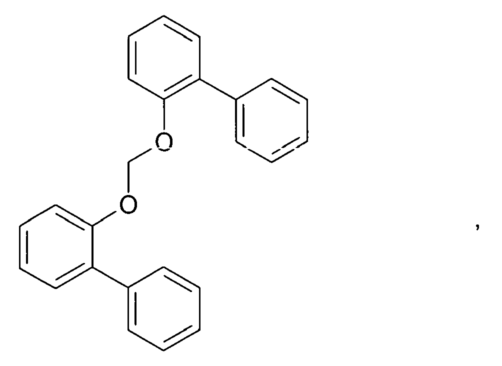 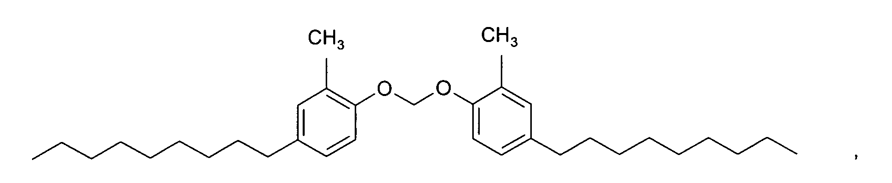 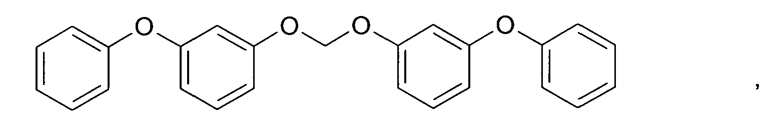 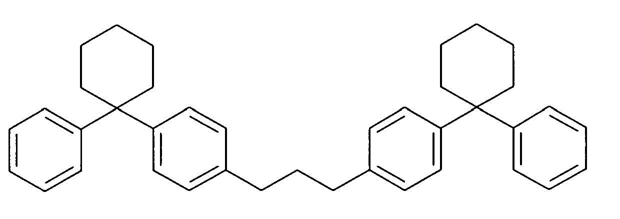 et
